# EUROPEAN PATENT SPECIFICATION

(11) **EP 2 176 412 B1**
(45) Date of publication and mention of the grant of the patent: **13.09.2017**
(21) Application number: 08768425.4
(22) Date of filing: 13.06.2008
(51) Int. Cl.: C12N 15/11, C12N 15/63, C12N 15/113

(54) **BACTERIAL MEDIATED TNF-ALPHA GENE SILENCING**
BAKTERIELL VERMITTELTES TNF-ALPHA GEN-SILENCING
SILENÇAGE DE GÈNE TNF-ALPHA MÉDIÉ PAR UNE BACTÉRIE

(30) Priority: 15.06.2007 US 934751 P; 04.01.2008 US 10028 P
(43) Date of publication of application: 21.04.2010
(73) Proprietor: Beth Israel Deaconess Medical Center, Boston, MA 02215 (US)
(72) Inventor: FRUEHAUF, Johannes, Newton, MA 02461 (US); LI, Chiang, Cambridge, MA 02141 (US)
(74) Representative: J A Kemp
(86) International application number: PCT/US2008/007384
(87) International publication number: WO 2008/156661

(56) References cited:
- EP-A1- 1 566 202
- WO-A-2006/007569
- WO-A-2006/066048
- KONG Y ET AL: "RNA interference as a novel and powerful tool in immunopharmacological research" INTERNATIONAL IMMUNOPHARMACOLOGY, ELSEVIER, AMSTERDAM, NL, vol. 7, no. 4, 21 February 2007 (2007-02-21), pages 417-426, XP005900656 ISSN: 1567-5769
- ZHANG Y ET AL: "Engineering Mucosal RNA Interference in Vivo" MOLECULAR THERAPY, ACADEMIC PRESS, SAN DIEGO, CA, US, vol. 14, no. 3, 1 September 2006 (2006-09-01), pages 336-342, XP005597047 ISSN: 1525-0016

## Description

### BACKGROUND

Gene silencing through RNAi (RNA-interference) by use of short interfering RNA (siRNA) has emerged as a powerful tool for molecular biology and holds the potential to be used for therapeutic gene silencing. Short hairpin RNA (shRNA) transcribed from small DNA plasmids within the target cell has also been shown to mediate stable gene silencing and achieve gene knockdown at levels comparable to those obtained by transfection with chemically synthesized siRNA (T. R. Brummelkamp, R. Bernards, R. Agami, Science 296, 550 (2002), P. J. Paddison, A. A. Caudiy, G. J. Hannon, PNAS 99, 1443 (2002)).

Possible applications of RNAi for therapeutic purposes are extensive and include silencing and knockdown of disease genes such as oncogenes or viral genes. One major obstacle for the therapeutic use of RNAi is the delivery of siRNA to the target cell (Zamore PD, Aronin N. Nature Medicine 9,(3):266-8 (2003)). In fact, delivery has been described as the major hurdle now for RNAi (Phillip Sharp, cited by Nature news feature, Vol 425, 2003, 10-12)

Two methods have been described which can be used in mouse models:
(1) Direct hydrodynamic intravenous injection of siRNA or shRNA-encoding plasmids: using this method, several authors have described application of RNAi against various conditions, *e.g.* hepatitis B (A.P. McCaffrey et al., Nat Biotechnol. 2003 Jun;21(6):639-44), fulminant hepatitis (E. Song, S. K. Lee, J. Wang, N. Ince, J. Min, J. Chen, P. Shankar, J. Lieberman. Nature Medicine 9, 347 (2003)), tumor xenograft (Spaenkuch B, et al. JNCI, 96(1): 862-72 (2004)), hepatic transgene expression (D. L. Lewis, J. E. Hagstrom, A. G. Loomis, J. A. Wolff, H. Hereijer, Nature Genetics, 32, 107 (2002), D. R. Sorensen DR, M. Leirdal, M. Sioud, JMB, 327, 761 (2003)). This method uses a high pressure and high volume injection (2.5 ml) into the mouse tail vein. The mechanism of siRNA/DNA uptake into the cells is not clear but probably mechanical damage to the vascular endothelial layer is developed into human application as it involves a massive volume charge and completely unknown mechanism of action.
(2) Direct injection into the target tissue (brain) of an siRNA encoding adenoviral vector (H. Xia, Q. Mao, H. L. Paulson, B. L. Davidson, Nat Biotechnol, 20, 1006 (2002)). This method showed silencing of transgene (GFP) expression in the brain tissues reached by the adenoviral vector. However, the area of silencing could not be predicted reliably. This method might be developed further and might become applicable for local, *e.g*. intratumoral injection. Viral vectors have been used widely for gene therapy purposes, but one lesson learned from gene therapy experiments is that viral spreading can be unpredictable at times and lead to unwanted side effects (Marshall E. Science 286(5448): 2244-5 (1999)). A new method is needed for the safe and predictable administration of interfering RNAs to mammals.

WO 2006/007569 discloses methods of preparing gene specific oligonucleotide libraries.

WO 2006/066048 discloses methods for the delivery of one or more small interfering RNAs (siRNAs) to a eukaryotic cell using a bacterium.

Kong *et al.* (2007) discloses the mechanisms of RNAi, methods of siRNA design and delivery and summarises recent research on the therapeutic potential of RNAi for immune diseases.

EP 1 566 202 discloses the use of an agent blocking the action of resistin for the treatment of Rheumatoid Arthritis.

### SUMMARY OF THE INVENTION

The present invention provides for an ex vivo method of delivering one or more siRNAs to mammalian cells, the method comprising introducing to said mammalian cells at least one invasive bacterium containing one or more siRNAs or one or more DNA molecules encoding one or more siRNAs, wherein the siRNAs are targeted to any one of the TNFα target sequences of SEQ ID NOs: 1, 4, 5, 7, 81, or 82, and wherein the siRNAs interfere with the mRNA of TNFα.

The present invention also provides for a method of regulating gene expression in mammalian cells, the method comprising introducing to said mammalian cells at least one invasive bacterium containing one or more siRNAs or one or more DNA molecules encoding one or more siRNAs, wherein the siRNAs are targeted to any one of the TNFα target sequences of SEQ ID NOs: 1, 4, 5, 7, 81, or 82, and wherein the siRNAs interfere with the mRNA of TNFα.

The present invention further provides for at least one invasive bacterium containing one or more siRNAs or one or more DNA molecules encoding one or more siRNAs, wherein the siRNAs are directed to any one of the TNFα target sequences of SEQ ID NOs: 1, 4, 5, 7, 81 or 82, and wherein the siRNAs interfere with the mRNA of TNFα for use in a method of treating or preventing an inflammatory disease or disorder in a mammal, the method comprising regulating the expression of at least one gene in a cell known to increase inflammation by introducing to the cells of the mammal said at least one invasive bacterium or said one or more DNA molecules.

In one embodiment of the invention, the expressed siRNAs direct the multienzyme complex RISC (RNA-induced silencing complex) of the cell to interact with the mRNA to be regulated. This complex degrades the mRNA. This causes the expression of the gene to be decreased or inhibited.

In one aspect of the invention the mammalian cell is a human cell.

In one embodiment of the invention, the mammal can be, but it not limited to, human, bovine, ovine, porcine, feline, buffalo, canine, goat, equine, donkey, deer, avian, bird, chicken and primate. The most preferred mammal is a human. In another embodiment the expressed siRNAs interfere with the mRNA of the gene to be regulated. In one aspect of this embodiment, the expressed siRNAs direct the multienzyme complex RISC (RNA-induced silencing complex) of the cell to interact with the mRNA to be regulated. This complex degrades the mRNA. This causes the expression of the gene to be decreased or inhibited.

In another embodiment of the invention, the cell is a colon cancer cell or a pancreatic cancer cell. In one aspect of this embodiment, the colon cancer cell is an SW 480 cell. In another aspect of this embodiment, the pancreatic cancer cell is a CAPAN-1 cell.

The invention also pertains to at least one invasive bacterium for use in a method of treating or preventing an inflammatory disease or disorder in a mammal, by regulating the expression of a gene or several genes known to cause an inflammatory disease or disorder in a cell, by introducing at least one bacterium to the cell. The bacterium contains one or more siRNAs or one or more DNA molecules encoding one or more siRNAs. The gene to be regulated is TNFα. Preferably, the regulation of the
gene decreases or lessens the expression and/or activity of the gene known to cause an inflammatory disease or disorder.

In one embodiment of the invention, the mammal can be, but it not limited to, human, bovine, ovine, porcine, feline, buffalo, canine, goat, equine, donkey, deer, avian, bird, chicken and primate. The most preferred mammal is a human. In another embodiment of this method the expressed siRNAs interfere with the mRNA of the gene or genes to be regulated. In one aspect of this embodiment, the expressed siRNAs direct the multienzyme complex RISC (RNA-induced silencing complex) of the cell to interact with the mRNA to be regulated. This complex degrades the mRNA. This causes the expression of the gene to be decreased or inhibited.

In another embodiment the inflammatory disease or disorder is inflammatory bowel disease. In another embodiment of this method, the inflammatory disease or disorder is Crohn's disease. In another embodiment, the inflammatory disease or disorder is ulcerative colitis. In another embodiment, the inflammatory disease or disorder is an allergy. In another embodiment, the cell is a gastrointestinal epithelial cell. In another embodiment, the cell is a macrophage cell.

In one embodiment of the invention, the bacterium is non-pathogenic or non-virulent. In another aspect of this embodiment, the bacterium is therapeutic. In another aspect of this embodiment, the bacterium is an attenuated strain selected from the group consisting of *Listeria, Shigella, Salmonella, E. coli,* and *Bifidobacteriae.* Optionally, the *Salmonella* strain is an attenuated strain of the *Salmonella typhimurium* species. Optionally, the *Salmonella typhimurium* strain is SL 7207 or VNP20009.

In another embodiment of the invention, the one or more DNA molecules encoding the one or more siRNAs are transcribed within the eukaryotic cell. In one aspect of this embodiment, the one or more siRNAs are transcribed within the eukaryotic cells as shRNAs. In another aspect of this embodiment, the one or more DNA molecules encoding the one or more siRNAs contains an RNA-polymerase III promoter. Optionally, the RNA polymerase III promoter is a U6 promoter or an H1 promoter.

In another embodiment of the invention, the one or more DNA molecules encoding one or more siRNAs are transcribed within the bacterium. In one aspect of this embodiment, the one or more DNA molecules contain a prokaryotic promoter. Optionally, the prokaryotic promoter is a T7 promoter.

In another embodiment of the invention, the one or more DNA molecules are introduced to the eukaryotic cell through type III export or bacterial lysis. In one aspect of this embodiment, the bacterial lysis is triggered by the addition of an intracellular active antibiotic. Optionally, the antibiotic is tetracycline. In another aspect of this embodiment, the bacterial lysis is triggered through bacterial metabolic attenuation. Optionally, the metabolic attenuation is auxotrophy.

The invention also pertains to a bacterium containing one or more siRNAs or one or more DNA molecules encoding one or more siRNAs.

In one embodiment of the invention, the bacterium is a non-pathogenic or a non-virulent bacterium. In another aspect of this embodiment, the bacterium is a therapeutic bacterium.

In another embodiment of the invention, the bacterium is an attenuated strain or derivative thereof selected from, but not limited to Yersinia, Rickettsia, Legionella, Brucella, Mycobacterium, Helicobacter, Haemophilus, Coxiella, Chlamydia, Neisseria, Burkolderia, Bordetella, Borrelia, Listeria, Shigella, Salmonella, Staphylococcus, Streptococcus, Porphyromonas, Treponema, Vibrio, E. coli, and Bifidobacteriae. Optionally, the Yersinia strain is an attenuated strain of the Yersinia pseudotuberculosis species. Optionally, the Yersinia strain is an attenuated strain of the Yersinia enterocolitica species. Optionally, the Rickettsia strain is an attenuated strain of the Rickettsia coronii species. Optionally, the Legionella strain is an attenuated strain of the Legionella pneumophilia species. Optionally, the Mycobacterium strain is an attenuated strain of the Mycobacterium tuberculosis species. Optionally, the Mycobacterium strain is an attenuated strain of the Mycobacterium bovis BCG species. Optionally, the Helicobacter strain is an attenuated strain of the Helicobacter pylori species. Optionally, the Coxiella strain is an attenuated strain of Coxiella burnetti. Optionally, the Haemophilus strain is an attenuated strain of the Haemophilus influenza species. Optionally, the Chlamydia strain is an attenuated strain of the Chlamydia trachomatis species. Optionally, the Chlamydia strain is an attenuated strain of the Chlamydia pneumoniae species. Optionally, the Neisseria strain is an attenuated strain of the Neisseria gonorrheae species. Optionally, the Neisseria strain is an attenuated strain of the Neisseria meningitides species. Optionally, the Burkolderia strain is an attenuated strain of the Burkolderia cepacia species. Optionally, the Bordetella strain is an attenuated strain of the Bordetella pertussis species. Optionally, the Borrelia strain is an attenuated strain of the Borrelia hermisii species. Optionally, the Listeria strain is an attenuated strain of the Listeria monocytogenes species. Optionally, the Listeria strain is an attenuated strain of the Listeria ivanovii species. Optionally, the Salmonella strain is an attenuated strain of the Salmonella enterica species. Optionally, the Salmonella strain is an attenuated strain of the Salmonella typhimurium species. Optionally, the Salmonella typhimurium strain is SL 7207 or VNP20009. Optionally, the Staphylococcus strain is an attenuated strain of the Staphylococcus aureus species. Optionally, the Streptococcus strain is an attenuated strain of the Streptococcus pyogenes species. Optionally, the Streptococcus strain is an attenuated strain of the Streptococcus mutans species. Optionally, the Streptococcus strain is an attenuated strain of the Streptococcus salivarius species. Optionally, the Streptococcus strain is an attenuated strain of the Streptococcus pneumonia species. Optionally, the Porphyromonas strain is an attenuated strain of the Porphyromonas gingivalis species. Optionally, the Pseudomonas strain is an attenuated strain of the Pseudomonas aeruginosa species. Optionally, the Treponema strain is an attenuated strain of the Treponema pallidum species. Optionally, the Vibrio strain is an attenuated strain of the Vibrio cholerae species. In one embodiment of the present invention, the prokaryotic vector contains a DNA encoding one or more siRNAs and an RNA-polymerase III compatible promoter or a prokaryotic promoter.

In one embodiment of the invention, the RNA polymerase III promoter is a U6 promoter or an H1 promoter. In another embodiment of this vector of the invention, the prokaryotic promoter is a T7 promoter.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

In the case of conflict, the present specification, including definitions, will control. In addition, the materials, methods, and examples are illustrative only and are not intended to be limiting.

Other features and advantages of the invention will be apparent from the following detailed description and claims.

### BRIEF DESCRIPTION OF THE DRAWINGS

Figure 1A shows micrographs of invasion of SL 7207 into SW 480 cells. Figure 1B shows FACS analysis of a knockdown of green fluorescent protein expression in CRL 2583 cells. Figure 1C shows micrographs showing loss of fluorescence in CRL 2583 cells.
Figure 2A shows Western blots of *k-Ras* and *β-catenin* in SW 480 cells. Figure 2B is a series of bar charts showing viability of SW 480 cells under various treatment regimens. Figure 2C shows photographs of tumorigenicity in nude mice injected with SW 480 cells transfected with various siRNAs.
Figure 3A shows micrographs of transgenic mouse liver sections. Figure 3B shows flow cytometry measurements of hepatocyte and splenocyte suspensions.
Figure 4 shows Western blots of *k-Ras* and *β-catenin* from SW 480 cells transfected with silencer plasmid.
Figure 5 shows micrographs of histochemical staining of liver sections of mice showing changes in GFP expression levels.

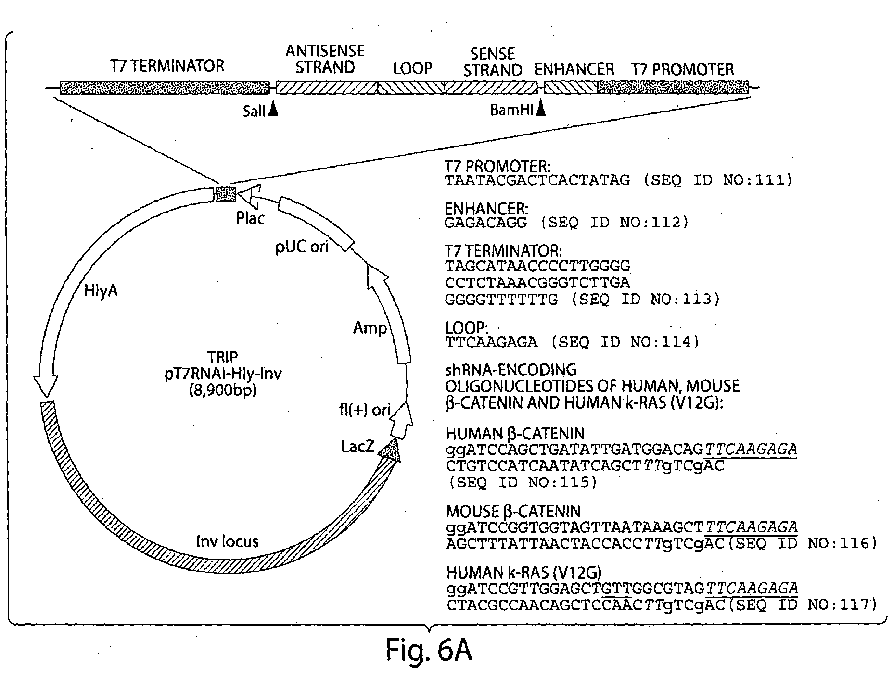

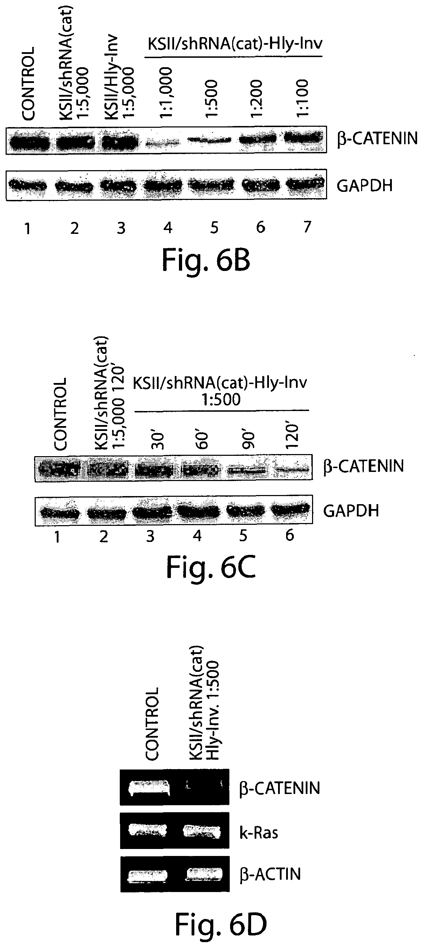

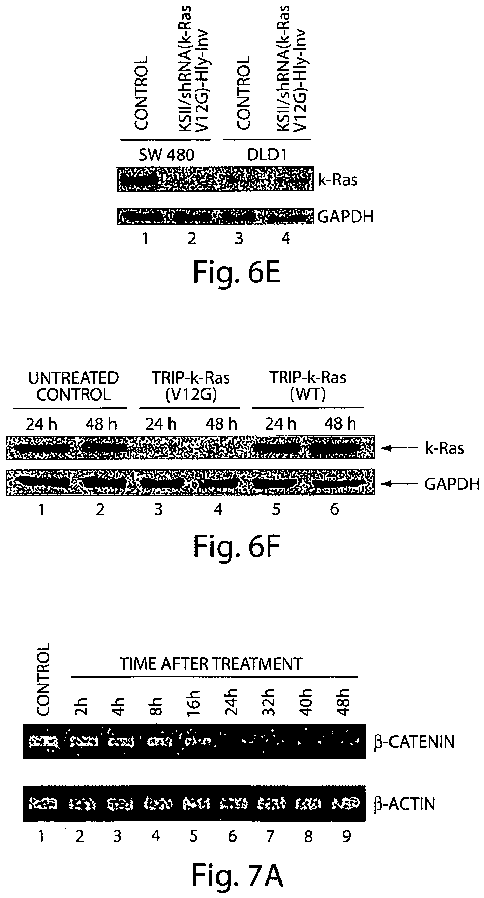

Figure 6A is a schematic showing the Transkingdom RNA Interference Plasmid (TRIP). Figure 6B is a photograph showing an immunoblot of *β-catenin* from SW 480 cells transfected with TRIP. Figure 6C is a photograph showing an immunoblot of *β-catenin* from SW 480 cells transfected with TRIP for exposure time from 30 to 120 minutes. Figure 6D shows an RT-PCR photograph of *β-catenin* and *k-Ras* mRNA from SW 480 cells transfected with TRIP. Figure 6E is a photograph showing an immunoblot of *k-Ras* in SW 480 and DLD1 cells following transfection with a TRIP against mutant *k-Ras* (GGT→GTT at codon 12) mutant *k-Ras* (GGC→GAC at codon 13. Figure 6F is a photograph showing an immunoblot of SW 480 cells transfected with a TRIP against wild type *k-Ras.*
Figure 7A is a photograph of RT-PCR showing *β-catenin* silencing following treatment with *E. coli* expressed shRNAs. Figure 7B is schematic showing specific cleavage sites in *β-catenin.* Figure 7C is a photograph of a 5'-RACE-PCR showing specific cleavage products. Figure 7D is a photograph of a blot showing the mRNA expression of various genes.

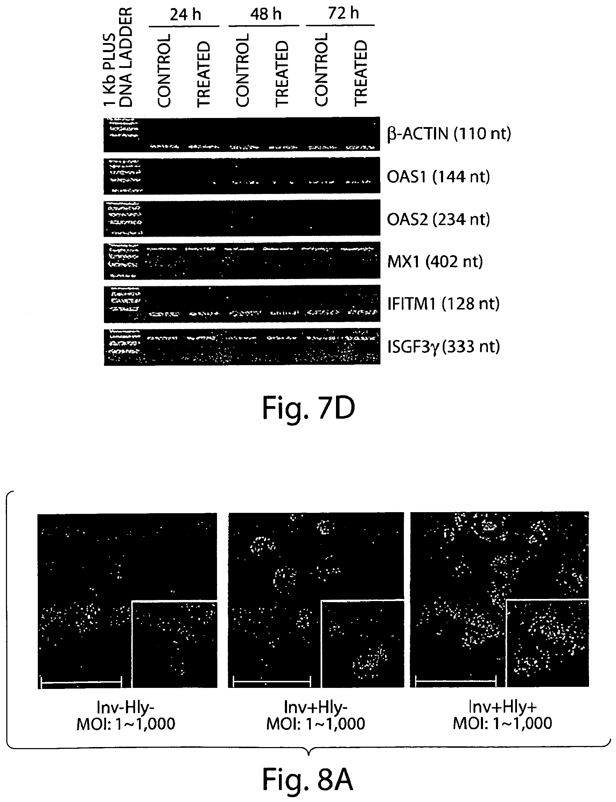

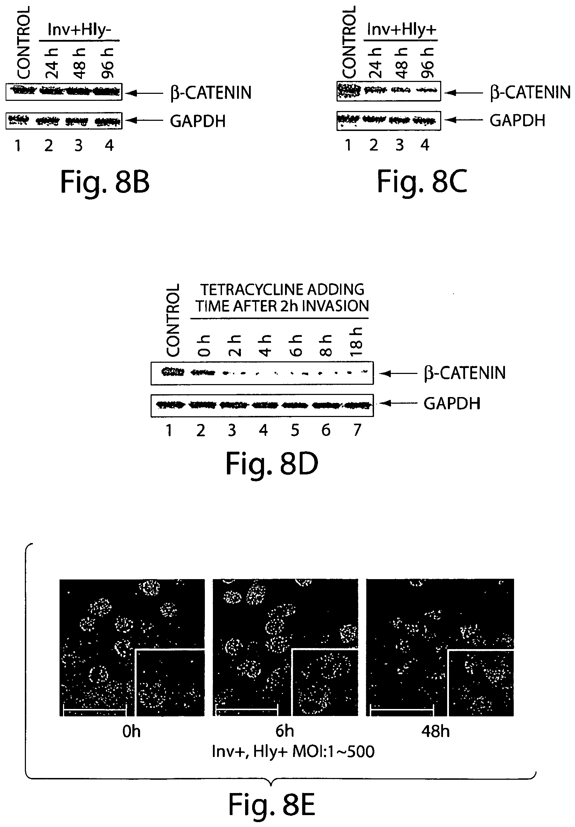

Figure 8A is a photograph of cellular staining showing that both Inv and Hly are required for bacterial entry. Figure 8B is a photograph of an RNA blot showing that TRIP lacking Hly is unable to induce knockdown of a target gene. Figure 8C is a photograph of an RNA blot showing that both Inv and Hly are required to facilitate efficient transkingdom iRNA. Figure 8D is a photograph of an RNA blot showing the effect of delayed addition of tetracycline on gene silencing. Figure 8E is a photograph of cellular staining showing lack of significant bacterial replication in the absence of antibiotics beyond 2 h incubation.

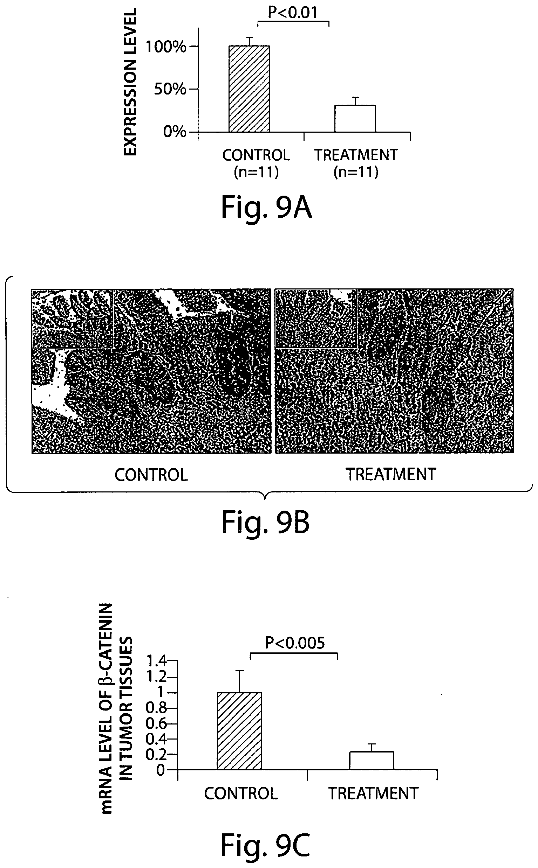

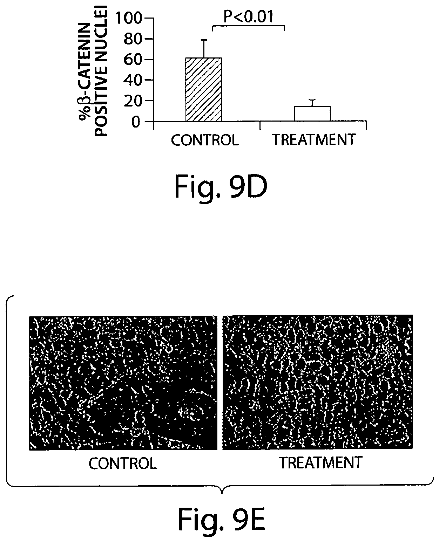

Figure 9A is a graph showing that oral administration of *E*. *coli* expressing shRNA against *β-catenin* in mice leads to significant reduction of *β*-*catenin* expression in the intestinal epithelium. Figure 9B is a photograph of immunohistochemistry staining of intestinal epithelium with or without treatment. Figure 9C is a graph showing a decrease in *β-catenin* mRNA expression following treatment. Figure 9D is a graph showing a decrease *in β-catenin* protein expression following treatment. Figure 9E is a photograph of immunohistochemistry staining showing decrease in *β-catenin* protein expression following treatment.
Figure 10 is a photograph of immunohistochemistry staining showing that GAPDH expression is not altered by E. coli expressing shRNA against *β-catenin* after oral dosing in mice.
Figure 11 is a graph illustrating reduction of TNF-α expression.

### DETAILED DESCRIPTION OF THE INVENTION

The invention pertains to ex vivo methods of delivering small interfering RNAs (siRNAs) to eukaryotic cells using non-pathogenic or therapeutic strains of bacteria. The bacteria deliver RNA encoding DNA or RNA, itself, to effect RNA interference (RNAi). The interfering RNA of the invention regulates gene expression in eukaryotic cells. It silences or knocks down genes of interest inside target cells. The interfering RNA directs the cell-owned multienzyme-complex RISC (RNA-induced silencing complex) to the mRNA of the gene to be silenced. Interaction of RISC and mRNA results in degradation of the mRNA. This leads to effective post-transcriptional silencing of the gene of interest. This method is referred to as Bacteria Mediated Gene Silencing (BMGS).

Bacterial delivery is more attractive than viral delivery as it can be controlled by use of antibiotics and attenuated bacterial strains which are unable to multiply. Also, bacteria are much more accessible to genetic manipulation which allows the production of vector strains specifically tailored to certain applications. In one embodiment of the invention, the methods are used to create bacteria which cause RNAi in a tissue specific manner.

The siRNA is either introduced into the target cell directly or by transfection or can be transcribed within the target cell as hairpin-structured dsRNA (shRNA) from specific plasmids with RNA-polymerase III compatible promoters (U6, H1) (P. J. Paddison, A. A. Caudiy, G. J. Hannon, PNAS 99, 1443 (2002), T. R. Brummelkamp, R. Bernards, R. Agami, Science 296, 550 (2002)).

Liberation of siRNA encoding plasmid from the intracellular bacteria occurs through active mechanisms. One mechanism involves the type III export system in *S. typhimuriumm,* a specialized multiprotein complex spanning the bacterial cell membrane whose functions include secretion of virulence factors to the outside of the cell to allow signaling towards the target cell, but which can also be used to deliver antigens into target cells. (Rüssmann H. Int J Med Microbiol, 293:107-12 (2003)) or through bacterial lysis and liberation of bacterial contents into the cytoplasm. The lysis of intracellular bacteria is triggered through addition of an intracellularly active antibiotic (tetracycline) or occurs naturally through bacterial metabolic attenuation (auxotrophy). After liberation of the eukaryotic transcription plasmid, shRNA or siRNA are produced within the target cell and trigger the highly specific process of mRNA degradation, which results in silencing of the targeted gene.

The non-virulent bacteria of the invention have invasive properties and may enter a mammalian host cell through various mechanisms. In contrast to uptake of bacteria by professional phagocytes, which normally results in the destruction of the bacterium within a specialized lysosome, invasive bacteria strains have the ability to invade non-phagocytic host cells. Naturally occurring examples of such bacteria are intracellular pathogens such as *Yersinia, Rickettsia, Legionella, Brucella, Mycobacterium, Helicobacter, Coxiella, Chlamydia, Neisseria, Burkolderia, Bordetella, Borrelia, Listeria, Shigella, Salmonella, Staphylococcus, Streptococcus, Porphyromonas, Treponema,* and *Vibrio,* but this property can also be transferred to other bacteria such as *E. coli* and *Bifidobacteriae,* including probiotics through transfer of invasion-related genes (P. Courvalin, S. Goussard, C. Grillot-Courvalin, C.R.Acad.Sci.Paris 318,1207 (1995)). In other embodiments of the invention, bacteria used to deliver interfering RNAs to host cells include *Shigella flexneri* (D. R. Sizemore, A. A. Branstrom, J. C. Sadoff, Science 270, 299 (1995)), invasive *E*. *coli* (P. Courvalin, S. Goussard, C. Grillot-Courvalin, C.R.Acad.Sci.Paris 318,1207 (1995), C. Grillot-Courvalin, S. Goussard, F. Huetz, D. M. Ojcius, P. Courvalin, Nat Biotechnol 16, 862 (1998)), *Yersinia enterocolitica* (A. Al-Mariri A, A. Tibor, P. Lestrate, P. Mertens, X. De Bolle, J. J. Letesson Infect Immun 70, 1915 (2002)) and *Listeria monocytogenes* (M. Hense, E. Domann, S. Krusch, P. Wachholz, K. E. Dittmar, M. Rohde, J. Wehland, T. Chakraborty, S. Weiss, Cell Microbiol 3, 599 (2001), S. Pilgrim, J. Stritzker, C. Schoen, A. Kolb-Mäurer, G. Geginat, M. J. Loessner, I. Gentschev, W. Goebel, Gene Therapy 10, 2036 (2003)). Any invasive bacterium is useful for DNA transfer into eukaryotic cells (S. Weiss, T. Chakraborty, Curr Opinion Biotechnol 12, 467 (2001)).

BMGS is performed using the naturally invasive pathogen *Salmonella typhimurium.* In one aspect of this embodiment, the strains of *Salmonella typhimurium* include SL 7207 and VNP20009 (S. K. Hoiseth, B. A. D. Stocker, Nature 291, 238 (1981); Pawelek JM, Low KB, Bermudes D. Cancer Res. 57(20):4537-44 (Oct. 15 1997)). In another embodiment of the invention, BMGS is performed using attenuated *E. coli.* In one aspect of this embodiment, the strain of *E. coli* is BM 2710 (C. Grillot-Courvalin, S. Goussard, F. Huetz, D. M. Ojcius, P. Courvalin, Nat Biotechnol 16, 862 (1998)). In another aspect of this embodiment, the BM 2710 strain is engineered to possess cell-invading properties through an invasion plasmid. In one aspect of the invention, this plasmid is pGB2inv-hly.

A double "trojan horse" technique is also used with an invasive and auxotrophic bacterium carrying a eukaryotic transcription plasmid. This plasmid is, in turn, transcribed by the target cell to form a hairpin RNA structure that triggers the intracellular process of RNAi. Significant gene silencing of a variety of genes is induced.

The invention also pertains to a variation of the described method, termed Bacteria Transcribed Gene Silencing (BTGS). In this aspect of the invention, siRNA is directly produced by the invasive bacteria as opposed to the target cell. A transcription plasmid controlled by a prokaryotic promoter (e.g. T7) is inserted into the carrier bacteria through standard transformation protocols. siRNA is produced within the bacteria and is liberated within the mammalian target cell after bacterial lysis triggered either by auxotrophy or by timed addition of antibiotics.

The invention also encompasses a prokaryotic shRNA-encoding transcription plasmid for use with invasive bacteria to perform Bacteria-Transcribed Gene Silencing (BTGS). These plasmids are used to screen different cancer-related targets in transgenic as well as wild type animals for therapeutic experiments.

The RNAi methods of the invention, including BMGS and BTGS are also used to create cancer-preventing "probiotic bacteria" for use, especially with the target of GI tract or liver.

The RNAi methods of the disclosure including BMGS and BTGS are used as therapy against inflammatory conditions, *e.g*. inflammatory bowel disease (IBD) or colitis. These methods are used to silence or knockdown non-cancer gene targets (viral genes, for treatment and prevention of hepatitis B, C; inflammatory genes, for treatment and prevention of inflammatory bowel disease) and others.

The invention also pertains to at least one invasive bacterium for use in a method of treating or preventing an inflammatory disease or disorder in a mammal, by regulating the expression of a gene or several genes known to cause an inflammatory disease or disorder in a cell, by introducing at least one bacterium to the cell. The bacterium contains one or more siRNAs or one or more DNA molecules encoding one or more siRNAs.

In one embodiment of the invention, the mammal can be, but it not limited to, human, bovine, ovine, porcine, feline, buffalo, canine, goat, equine, donkey, deer, avian, bird, chicken and primate. The most preferred mammal is a human. In another embodiment the expressed siRNAs interfere with the mRNA of the gene or genes to be regulated. In one aspect of this embodiment, the expressed siRNAs direct the multienzyme complex RISC (RNA-induced silencing complex) of the cell to interact with the mRNA to be regulated. This complex degrades the mRNA. This causes the expression of the gene to be decreased or inhibited.

In another embodiment, the inflammatory disease or disorder is inflammatory bowel disease. In another embodiment, the inflammatory disease or disorder is Crohn's disease. In another embodiment, the inflammatory disease or disorder is ulcerative colitis. In another embodiment, the inflammatory disease or disorder is an allergy. In another embodiment, the cell is a gastrointestinal epithelial cell. In another embodiment, the cell is a macrophage cell.

The gene to be regulated is TNFα. Preferably, the regulation of the gene decreases or lessens the expression and/or activity of the gene known to cause an inflammatory disease or disorder.

In another aspect of this embodiment, the TNFα target gene sequence is SEQ ID NO:s 1, 4, 5, 7, 81 or 82. The sequences in Table 1 are cross-species target sequences as they are capable of silencing the TNFα in human and mouse.

**Table 1.**

| | |
|---|---|
| CCCGAGTGACAAGCCTGTAGC | (SEQ ID NO: 1) |
| CCGAGTGACAAGCCTGTAGCC | (SEQ ID NO: 2) |
| CGAGTGACAAGCCTGTAGCCC | (SEQ ID NO: 3) |
| GAGTGACAAGCCTGTAGCCCA | (SEQ ID NO: 4) |
| AGTGACAAGCCTGTAGCCCAT | (SEQ ID NO: 5) |
| ATGTGCTCCTCACCCACACCA | (SEQ ID NO: 6) |
| AGGTCAACCTCCTCTCTGCCA | (SEQ ID NO: 7) |
| GGTCTTCCAGCTGGAGAAGGG | (SEQ ID NO: 8) |
| AGATGAATGTATTTATTTGGG | (SEQ ID NO: 9) |

The sequences in Table 2 are target sequences as they are capable of silencing the TNFα in human.

**Table 2.**

| | |
|---|---|
| CCTGATCGTGGCAGGCGCCAC | (SEQ ID NO: 10) |
| GGCAGGCGCCACCACGCTCTT | (SEQ ID NO: 11) |
| GCATCGCCGTCTCCTACCAGA | (SEQ ID NO: 12) |
| GGTGACCGACTCAGCGCTGAG | (SEQ ID NO: 13) |
| ACTCAGCGCTGAGATCAATCG | (SEQ ID NO: 14) |
| CTCAGCGCTGAGATCAATCGG | (SEQ ID NO: 15) |
| TCAGCGCTGAGATCAATCGGC | (SEQ ID NO: 16) |
| CAGCGCTGAGATCAATCGGCC | (SEQ ID NO: 17) |
| CTGAGATCAATCGGCCCGACT | (SEQ ID NO: 18) |
| TGAGATCAATCGGCCCGACTA | (SEQ ID NO: 19) |
| GAGATCAATCGGCCCGACTAT | (SEQ ID NO: 20) |
| AGATCAATCGGCCCGACTATC | (SEQ ID NO: 21) |
| GATCAATCGGCCCGACTATCT | (SEQ ID NO: 22) |
| ATCAATCGGCCCGACTATCTC | (SEQ ID NO: 23) |
| TCAATCGGCCCGACTATCTCG | (SEQ ID NO: 24) |
| CAATCGGCCCGACTATCTCGA | (SEQ ID NO: 25) |
| AATCGGCCCGACTATCTCGAC | (SEQ ID NO: 26) |
| ATCGGCCCGACTATCTCGACT | (SEQ ID NO: 27) |
| TCGGCCCGACTATCTCGACTT | (SEQ ID NO: 28) |
| CGGCCCGACTATCTCGACTTT | (SEQ ID NO: 29) |
| GCCCGACTATCTCGACTTTGC | (SEQ ID NO: 30) |
| CCCGACTATCTCGACTTTGCC | (SEQ ID NO: 31) |
| GACTATCTCGACTTTGCCGAG | (SEQ ID NO: 32) |
| ACTATCTCGACTTTGCCGAGT | (SEQ ID NO: 33) |
| CTATCTCGACTTTGCCGAGTC | (SEQ ID NO: 34) |
| TATCTCGACTTTGCCGAGTCT | (SEQ ID NO: 35) |
| GGAGGACGAACATCCAACCTT | (SEQ ID NO: 36) |
| TAGGGTCGGAACCCAAGCTTA | (SEQ ID NO: 37) |
| GGGTCGGAACCCAAGCTTAGA | (SEQ ID NO: 38) |
| GGTCGGAACCCAAGCTTAGAA | (SEQ ID NO: 39) |
| CTGTAATCGCCCTACTATTCA | (SEQ ID NO: 40) |
| TGTAATCGCCCTACTATTCAG | (SEQ ID NO: 41) |
| GCCCTACTATTCAGTGGCGAG | (SEQ ID NO: 42) |
| CCCTACTATTCAGTGGCGAGA | (SEQ ID NO: 43) |
| CCTACTATTCAGTGGCGAGAA | (SEQ ID NO: 44) |
| CTACTATTCAGTGGCGAGAAA | (SEQ ID NO: 45) |

The sequences in Table 3 are target sequences as they are capable of silencing the TNFα in mouse.

**Table 3.**

| | |
|---|---|
| AGAAAGCATGATCCGCGACGT | (SEQ ID NO: 46) |
| AAGCATGATCCGCGACGTGGA | (SEQ ID NO: 47) |
| AGCATGATCCGCGACGTGGAA | (SEQ ID NO: 48) |
| CATGATCCGCGACGTGGAACT | (SEQ ID NO: 49) |
| ATGATCCGCGACGTGGAACTG | (SEQ ID NO: 50) |
| ATCCGCGACGTGGAACTGGCA | (SEQ ID NO: 51) |
| AAGCCTGTAGCCCACGTCGTA | (SEQ ID NO: 52) |
| CCTGTAGCCCACGTCGTAGCA | (SEQ ID NO: 53) |
| CCCACGTCGTAGCAAACCACC | (SEQ ID NO: 54) |
| GAGCCAGCGCGCCAACGCCCT | (SEQ ID NO: 55) |
| CAACGCCCTCCTGGCCAACGG | (SEQ ID NO: 56) |
| CAACTAGTGGTGCCAGCCGAT | (SEQ ID NO: 57) |
| AACTAGTGGTGCCAGCCGATG | (SEQ ID NO: 58) |
| CCAGCCGATGGGTTGTACCTT | (SEQ ID NO: 59) |
| ACCCACACCGTCAGCCGATTT | (SEQ ID NO: 60) |
| CCCACACCGTCAGCCGATTTG | (SEQ ID NO: 61) |
| CCACACCGTCAGCCGATTTGC | (SEQ ID NO: 62) |
| CACACCGTCAGCCGATTTGCT | (SEQ ID NO: 63) |
| CACCGTCAGCCGATTTGCTAT | (SEQ ID NO: 64) |
| ACCGTCAGCCGATTTGCTATC | (SEQ ID NO: 65) |
| CAAGTACTTAGACTTTGCGGA | (SEQ ID NO: 66) |
| AAGTACTTAGACTTTGCGGAG | (SEQ ID NO: 67) |
| AGTACTTAGACTTTGCGGAGT | (SEQ ID NO: 68) |
| GACTTTGCGGAGTCCGGGCAG | (SEQ ID NO: 69) |
| GAGTCCGGGCAGGTCTACTTT | (SEQ ID NO: 70) |
| GGGAGCCGTGACTGTAATCGC | (SEQ ID NO: 71) |
| GGAGCCGTGACTGTAATCGCC | (SEQ ID NO: 72) |
| GAGCCGTGACTGTAATCGCCC | (SEQ ID NO: 73) |
| AGCCGTGACTGTAATCGCCCT | (SEQ ID NO: 74) |
| GCCGTGACTGTAATCGCCCTA | (SEQ ID NO: 75) |
| GACTGTAATCGCCCTACGGGT | (SEQ ID NO: 76) |
| ACTGTAATCGCCCTACGGGTC | (SEQ ID NO: 77) |
| CTGTAATCGCCCTACGGGTCA | (SEQ ID NO: 78) |
| TGTAATCGCCCTACGGGTCAT | (SEQ ID NO: 79) |
| TAATCGCCCTACGGGTCATTG | (SEQ ID NO: 80) |
| AATCGCCCTACGGGTCATTGA | (SEQ ID NO: 81) |
| ATCGCCCTACGGGTCATTGAG | (SEQ ID NO: 82) |

In one embodiment to target these sequences, a Transkingdom RNA Interference Plasmid (TRIP) will incorporate a hairpin RNA expression cassette encoding short hairpin RNA under the control of a T7 RNA polymerase promoter and terminator. In the design of these constructs, an algorithm was utilized to take into account some known difficulties with the development of siRNA, namely: (1) Exclusion of disqualifying properties (SNPs, interferon motifs); (2) Exclusion of the sequence if there was homology in ref seq (19/21, >17 contiguous to any other genes) and (3) Exclusion of the sequence if there were significant miRNA seed type matches. After screening and rejection of all possible siRNA target sequences for human beta catenin according to these criteria, the remaining ones were checked for conservation in the other species as indicated in this list.

In one aspect of this embodiment, the DNA insert comprises one or more of the following constructs, each of which contains a TNFα target sequence, a hairpin sequence and BamH1 and Sal1 restriction sites to facilitate incorporation into the hairpin RNA expression cassette of the TRIP plasmid:

| BamHI | sense (19bp) | loop | antisense (21bp) | SaII |
|---|---|---|---|---|
| | **SEQ ID NO: 41** | | | |
| 5'-GATCC | CGAGTGACAAGCCTGTAGC | TTCAAGAGA | GCTACAGGCTTGTCACTCGGG | G-3' (SEQ ID NO:83) |
| 3'-G | GCTCACTGTTCGGACATCG | AAGTTCTCT | CGATGTCCGAACAGTGAGCCC | CAGCT-5' (SEQ ID NO:84) |
| | | | | |
| | **SEQ ID NO: 44** | | | |
| 5'-GATCC | GTGACAAGCCTGTAGCCCA | TTCAAGAGA | TGGGCTACAGGCTTGTCACTC | G-3' (SEQ ID NO:85) |
| 3'-G | CACTGTTCGGACATCGGGT | AAGTTCTCT | ACCCGATGTCCGAACAGTGAG | CAGCT-5' (SEQ ID NO:86) |
| | | | | |
| | **SEQ ID NO: 45** | | | |
| 5'-GATCC | TGACAAGCCTGTAGCCCAT | TTCAAGAGA | ATGGGCTACAGGCTTGTCACT | G-3' (SEQ ID NO:87) |
| 3'-G | ACTGTTCGGACATCGGGTA | AAGTTCTCT | TACCCGATGTCCGAACAGTGA | CAGCT-5' (SEQ ID NO:88) |
| | | | | |
| | **SEQ ID NO: 47** | | | |
| 5'-GATCC | GTCAACCTCCTCTCTGCCA | TTCAAGAGA | TGGCAGAGAGGAGGTTGACCT | G-3' (SEQ ID NO:89) |
| 3'-G | CAGTTGGAGGAGAGACGGT | AAGTTCTCT | ACCGTCTCTCCTCCAACTGGA | CAGCT-5' (SEQ ID NO:90) |
| **90** | | | | |
| | **SEQ ID NO: 121** | | | |
| 5'-GATCC | TCGCCCTACGGGTCATTGA | TTCAAGAGA | TCAATGACCCGTAGGGCGATT | G-3' (SEQ ID NO:91) |
| 3'-G | AGCGGGATGCCCAGTAACT | AAGTTCTCT | AGTTACTGGGCATCCCGCTAA | CAGCT-5' (SEQ ID NO:92) |
| | | | | |
| | **SEQ ID NO: 122** | | | |
| 5'-GATCC | CGCCCTACGGGTCATTGAG | TTCAAGAGA | CTCAATGACCCGTAGGGCGAT | G-3' (SEQ ID NO:93) |
| 3'-G | GCGGGATGCCCAGTAACTC | AAGTTCTCT | GAGTTACTGGGCATCCCGCTA | CAGCT-5' (SEQ ID NO:94) |

The RNAi methods of the invention, including BMGS and BTGS are used to create transient "knockdown" genetic animal models as opposed to genetically engineered knockout models to discover gene functions. The methods are also used as *in vitro* transfection tool for research and drug development

These methods use bacteria with desirable properties (invasiveness, attenuation, steerability) for example, *Bifidobacteria* and *Listeria,* are used to perform BMGS and BTGS. Invasiveness as well as eukaryotic or prokaryotic transcription of one or several shRNA is conferred to a bacterium using plasmids.

The RNAi methods of the disclosure including BMGS and BTGS are used for delivery of gene silencing to the gut and colon, and for oral application for use in a method of treating various diseases, namely colon cancer treatment and prevention. In another aspect of this embodiment, delivery of gene silencing is extra-intestinal.

### 1. Bacteria Delivering RNA to Eukaryotic Cells

According to the invention, any invasive bacterium which is capable of delivering a molecule, *e.g.,* an RNA molecule, into the cytoplasm of a target cell, such as by traversing the membrane and entering the cytoplasm of a cell, can be used to deliver RNA to such cells.

As used herein, the term "invasive" when referring to a microorganism, *e.g.,* a bacterium, refers to a microorganism which is capable of delivering at least one molecule, *e.g.,* an RNA or RNA-encoding DNA molecule, to a target cell. An invasive microorganism can be a microorganism which is capable of traversing a cell membrane, thereby entering the cytoplasm of said cell, and delivering at least some of its content, *e.g.,* RNA or RNA-encoding DNA, into the target cell. The process of delivery of the at least one molecule into the target cell preferably does not significantly modify the invasion apparatus.

The microorganism is a bacterium. A preferred invasive bacterium is a bacterium which is capable of delivering at least one molecule, *e.g.,* an RNA or RNA-encoding DNA molecule, to a target cells, such as by entering the cytoplasm of a eukaryotic cell. Preferred invasive bacteria are live bacteria, *e.g.,* live invasive bacteria.

Invasive microorganisms include microorganisms that are naturally capable of delivering at least one molecule to a target cell, such as by traversing the cell membrane, *e.g.*, a eukaryotic cell membrane, and entering the cytoplasm, as well as microorganisms which are not naturally invasive and which have been modified, *e.g.,* genetically modified, to be invasive. In another preferred embodiment, a microorganism which is not naturally invasive can be modified to become invasive by linking the bacterium to an "invasion factor", also termed "entry factor" or "cytoplasm-targeting factor". As used herein, an "invasion factor" is a factor, *e.g.,* a protein or a group of proteins which, when expressed by a non-invasive bacterium, render the bacterium invasive. As used herein, an "invasion factor" is encoded by a "cytoplasm-targeting gene".

Naturally invasive microorganisms, *e.g.,* bacteria, may have a certain tropism, *i.e*., preferred target cells. Alternatively, microorganisms, *e.g.,* bacteria can be modified, *e.g.,* genetically, to mimic the tropism of a second microorganism.

Delivery of at least one molecule into a target cell can be determined according to methods known in the art. For example, the presence of the molecule, by the decrease in expression of an RNA or protein silenced thereby, can be detected by hybridization or PCR methods, or by immunological methods which may include the use of an antibody.

Determining whether a microorganism is sufficiently invasive for use in the invention may include determining whether sufficient RNA, was delivered to host cells, relative to the number of microorganisms contacted with the host cells. If the amount of RNA, is low relative to the number of microorganisms used, it may be desirable to further modify the microorganism to increase its invasive potential.

Bacterial entry into cells can be measured by various methods. Intracellular bacteria survive treatment by aminoglycoside antibiotics, whereas extracellular bacteria are rapidly killed. A quantitative estimate of bacterial uptake can be achieved by treating cell monolayers with the antibiotic gentamicin to inactivate extracellular bacteria, then by removing said antibiotic before liberating the surviving intracellular organisms with gentle detergent and determining viable counts on standard bacteriological medium. Furthermore, bacterial entry into cells can be directly observed, e.g., by thin-section-transmission electron microscopy of cell layers or by immunofluorescent techniques (Falkow et al. (1992) Annual Rev. Cell Biol. 8:333). Thus, various techniques can be used to determine whether a specific bacteria is capable of invading a specific type of cell or to confirm bacterial invasion following modification of the bacteria, such modification of the tropism of the bacteria to mimic that of a second bacterium.

Bacteria that can be used for delivering RNA according to the invention are preferably non-pathogenic. However, pathogenic bacteria can also be used, so long as their pathogenicity has been attenuated, to thereby render the bacteria non-harmful to a subject to which it is administered. As used herein, the term "attenuated bacterium" refers to a bacterium that has been modified to significantly reduce or eliminate its harmfulness to a subject. A pathogenic bacterium can be attenuated by various methods, set forth below.

Without wanting to be limited to a specific mechanism of action, the bacterium delivering the RNA into the eukaryotic cell can enter various compartments of the cell, depending on the type of bacterium. For example, the bacterium can be in a vesicle, *e.g.,* a phagocytic vesicle. Once inside the cell, the bacterium can be destroyed or lysed and its contents delivered to the eukaryotic cell. A bacterium can also be engineered to express a phagosome degrading enyzme to allow leakage of RNA from the phagosome. In some embodiments, the bacterium can stay alive for various times in the eukaryotic cell and may continue to produce RNA. The RNA or RNA-encoding DNA can then be released from the bacterium into the cell by, *e.g.,* leakage. In certain embodiments of the invention, the bacterium can also replicate in the eukaryotic cell. In a preferred embodiment, bacterial replication does not kill the host cell. The invention is not limited to delivery of RNA or RNA-encoding DNA by a specific mechanism and is intended to encompass methods and compositions permitting delivery of RNA or RNA-encoding DNA by a bacterium independently of the mechanism of delivery.

Set forth below are examples of bacteria which have been described in the literature as being naturally invasive (section 1.1), as well as bacteria which have been described in the literature as being naturally non-invasive bacteria (section 1.2), as well as bacteria which are naturally non-pathogenic or which are attenuated. Although some bacteria have been described as being non-invasive (section 1.2), these may still be sufficiently invasive for use according to the invention. Whether traditionally described as naturally invasive or non-invasive, any bacterial strain can be modified to modulate, in particular to increase, its invasive characteristics (*e.g.,* as described in section 1.3).

### 1.1 Naturally Invasive Bacteria

The particular naturally invasive bacteria employed in the present invention is not critical thereto. Examples of such naturally-occurring invasive bacteria include, but are not limited to, *Shigella spp., Salmonella spp., Listeria spp., Rickettsia spp.,* and enteroinvasive *Escherichia coli.*

The particular *Shigella* strain employed is not critical to the present invention. Examples of *Shigella* strains which can be employed in the present invention include *Shigella flexneri* 2a (ATCC No. 29903), *Shigella sonnei* (ATCC No. 29930), and *Shigella disenteriae* (ATCC No. 13313). An attenuated *Shigella* strain, such as *Shigella flexneri* 2a 2457T aroA virG mutant CVD 1203 (Noriega *et al. supra), Shigella flexneri* M90T icsA mutant (Goldberg et al. Infect. Immun., 62:5664-5668 (1994)), *Shigella flexneri* Y SFL114 aroD mutant (Karnell et al. Vacc., 10:167-174 (1992)), and *Shigella flexneri* aroA aroD mutant (Verma et al. Vacc., 9:6-9 (1991)) are preferably employed in the present invention. Alternatively, new attenuated *Shigella spp.* strains can be constructed by introducing an attenuating mutation either singularly or in conjunction with one or more additional attenuating mutations.

At least one advantage to *Shigella* RNA vaccine vectors is their tropism for lymphoid tissue in the colonic mucosal surface. In addition, the primary site of *Shigella* replication is believed to be within dendritic cells and macrophages, which are commonly found at the basal lateral surface of M cells in mucosal lymphoid tissues (reviewed by McGhee, J. R. et al. (1994) Reproduction, Fertility, & Development 6:369; Pascual, D. W. et al. (1994) Immunomethods 5:56). As such, *Shigella* vectors may provide a means to express antigens in these professional antigen presenting cells. Another advantage of *Shigella* vectors is that attenuated *Shigella* strains deliver nucleic acid reporter genes *in vitro* and *in vivo* (Sizemore, D. R. et al. (1995) Science 270:299; Courvalin, P. et al. (1995) Comptes Rendus de 1 Academie des Sciences Serie III-Sciences de la Vie-Life Sciences 318:1207; Powell, R. J. et al. (1996) In: Molecular approaches to the control of infectious diseases. F. Brown, E. Norrby, D. Burton and J. Mekalanos, eds. Cold Spring Harbor Laboratory Press, New York. 183; Anderson, R. J. et al. (1997) Abstracts for the 97th General Meeting of the American Society for Microbiology:E.). On the practical side, the tightly restricted host specificity of *Shigella* stands to prevent the spread of *Shigella* vectors into the food chain via intermediate hosts. Furthermore, attenuated strains that are highly attenuated in rodents, primates and volunteers have been developed (Anderson *et al.* (1997) *supra;* Li, A. et al. (1992) Vaccine 10:395; Li, A. et al. (1993) Vaccine 11:180; Kamell, A. et al. (1995) Vaccine 13:88; Sansonetti, P. J. and J. Arondel (1989) Vaccine 7:443; Fontaine, A. et al. (1990) Research in Microbiology 141:907; Sansonetti, P. J. et al. (1991) Vaccine 9:416; Noriega, F. R. et al. (1994) Infection & Immunity 62:5168; Noriega, F. R. et al. (1996) Infection & Immunity 64:3055; Noriega, F. R. et al. (1996) Infection & Immunity 64:23; Noriega, F. R. et al. (1996) Infection & Immunity 64:3055; Kotloff, K. L. et al. (1996) Infection & Immunity 64:4542). This latter knowledge will allow the development of well tolerated *Shigella* vectors for use in humans.

Attenuating mutations can be introduced into bacterial pathogens using non-specific mutagenesis either chemically, using agents such as N-methyl-N'-nitro-N-nitrosoguanidine, or using recombinant DNA techniques; classic genetic techniques, such as Tn10 mutagenesis, P22-mediated transduction, λ phage mediated crossover, and conjugational transfer; or site-directed mutagenesis using recombinant DNA techniques. Recombinant DNA techniques are preferable since strains constructed by recombinant DNA techniques are far more defined. Examples of such attenuating mutations include, but are not limited to:
(i) auxotrophic mutations, such as aro (Hoiseth et al. Nature, 291:238-239 (1981)), gua (McFarland et al. Microbiol. Path., 3:129-141 (1987)), nad (Park et al. J. Bact., 170:3725-3730 (1988), thy (Nnalue et al. Infect. Immun., 55:955-962 (1987)), and asd (Curtiss, *supra)* mutations;
(ii) mutations that inactivate global regulatory functions, such as cya (Curtiss et al. Infect. Immun., 55:3035-3043 (1987)), crp (Curtiss et al (1987), *supra*), phoP/phoQ (Groisman et al. Proc. Natl. Acad. Sci., USA, 86:7077-7081 (1989); and Miller et al. Proc. Natl. Acad. Sci., USA, 86:5054-5058 (1989)), phop^{c} (Miller et al. J. Bact., 172:2485-2490 (1990)) or ompR (Dorman et al. Infect. Immun., 57:2136-2140 (1989)) mutations;
(iii) mutations that modify the stress response, such as recA (Buchmeier et al. Mol. Micro., 7:933-936 (1993)), htrA (Johnson et al. Mol. Micro., 5:401-407 (1991)), htpR (Neidhardt et al. Biochem. Biophys. Res. Com., 100:894-900 (1981)), hsp (Neidhardt et al. Ann. Rev. Genet., 18:295-329 (1984)) and groEL (Buchmeier et al. Sci., 248:730-732 (1990)) mutations;
(iv) mutations in specific virulence factors, such as IsyA (Libby et al. Proc. Natl. Acad. Sci., USA, 91:489-493 (1994)), pag or prg (Miller et al (1990), *supra;* and Miller et al (1989), *supra*), iscA or virG (d'Hauteville et al. Mol. Micro., 6:833-841 (1992)), plcA (Mengaud et al. Mol. Microbiol., 5:367-72 (1991); Camilli et al. J. Exp. Med, 173:751-754 (1991)), and act (Brundage et al. Proc. Natl. Acad. Sci., USA, 90:11890-11894 (1993)) mutations;
(v) mutations that affect DNA topology, such as topA (Galan et al. Infect. Immun., 58:1879-1885 (1990));
(vi) mutations that disrupt or modify the cell cycle, such as min (de Boer et al. Cell, 56:641-649 (1989)).
(vii) introduction of a gene encoding a suicide system, such as sacB (Recorbet et al. App. Environ. Micro., 59:1361-1366 (1993); Quandt et al. Gene, 127:15-21 (1993)), nuc (Ahrenholtz et al. App. Environ. Micro., 60:3746-3751 (1994)), hok, gef, kil, or phlA (Molin et al. Ann. Rev. Microbiol., 47:139-166 (1993));
(viii) mutations that alter the biogenesis of lipopolysaccharide and/or lipid A, such as rFb (Raetz in Esherishia coli and Salmonella typhimurium, Neidhardt et al., Ed., ASM Press, Washington D.C. pp 1035-1063 (1996)), ga1E (Hone et al. J. Infect. Dis., 156:164-167 (1987)) and htrB (Raetz, *supra),* msbB (Reatz, *supra*)
(ix) introduction of a bacteriophage lysis system, such as lysogens encoded by P22 (Rennell et al. Virol, 143:280-289 (1985)), λ murein transglycosylase (Bienkowska-Szewczyk et al. Mol. Gen. Genet., 184:111-114 (1981)) or S-gene (Reader et al. Virol, 43:623-628 (1971)); and

The attenuating mutations can be either constitutively expressed or under the control of inducible promoters, such as the temperature sensitive heat shock family of promoters (Neidhardt *et al. supra*), or the anaerobically induced nirB promoter (Harborne et al. Mol. Micro., 6:2805-2813 (1992)) or repressible promoters, such as uapA (Gorfinkiel et al. J. Biol. Chem., 268:23376-23381 (1993)) or gcv (Stauffer et al. J. Bact., 176:6159-6164 (1994)).

The particular *Listeria* strain employed is not critical to the present invention. Examples of *Listeria* strains which can be employed in the present invention include *Listeria monocytogenes* (ATCC No. 15313). Attenuated *Listeria* strains, such as *L. monocytogenes* actA mutant (Brundage *et al. supra*) or *L. monocytogenes* plcA (Camilli et al. J. Exp. Med., 173:751-754 (1991)) are preferably used in the present invention. Alternatively, new attenuated *Listeria* strains can be constructed by introducing one or more attenuating mutations in groups (i) to (vii) as described for *Shigella spp.* above.

The particular *Salmonella* strain employed is not critical to the present invention. Examples of *Salmonella* strains which can be employed in the present invention include *Salmonella typhi* (ATCC No. 7251) and *S. typhimurium* (ATCC No. 13311). Attenuated *Salmonella* strains are preferably used in the present invention and include *S*. *typhi-aro*C-aroD (Hone et al. Vacc. 9:810 (1991) and *S. typhimurium*-aroA mutant (Mastroeni et al. Micro. Pathol. 13:477 (1992)). Alternatively, new attenuated *Salmonella* strains can be constructed by introducing one or more attenuating mutations as described fro *Shigella spp.* above.

The particular *Rickettsia* strain employed is not critical to the present invention. Examples of *Rickettsia* strains which can be employed in the present invention include *Rickettsia Rickettsiae* (ATCC Nos. VR149 and VR891), *Ricketsia prowaseckii* (ATCC No. VR233), *Rickettsia tsutsugamuchi* (ATCC Nos. VR312, VR150 and VR609), *Rickettsia mooseri* (ATCC No. VR144), *Rickettsia sibirica* (ATCC No. VR151), and *Rochalimaea quitana* (ATCC No. VR358). Attenuated *Rickettsia* strains are preferably used in the present invention and can be constructed by introducing one or more attenuating mutations in groups (i) to (vii) as described for *Shigella spp.* above.

The particular enteroinvasive *Escherichia* strain employed is not critical to the present invention. Examples of enteroinvasive *Escherichia* strains which can be employed in the present invention include *Escherichia* coli strains 4608-58, 1184-68, 53638-C-17, 13-80, and 6-81 (Sansonetti et al. Ann. Microbiol. (Inst. Pasteur), 132A:351-355 (1982)). Attenuated enteroinvasive *Escherichia* strains are preferably used in the present invention and can be constructed by introducing one or more attenuating mutations in groups (i) to (vii) as described for *Shigella spp.* above.

Furthermore, since certain microorganisms other than bacteria can also interact with integrin molecules (which are receptors for certain invasion factors) for cellular uptake, such microorganisms can also be used for introducing RNA into target cells. For example, viruses, *e.g.,* foot-and-mouth disease virus, echovirus, and adenovirus, and eukaryotic pathogens, *e.g., Histoplasma capsulatum* and *Leishmania major* interact with integrin molecules.

### 1.2 Less Invasive Bacteria

Examples of bacteria which can be used in the invention and which have been described in the literature as being non-invasive or at least less invasive than the bacteria listed in the previous section (1.1) include, but are not limited to, *Yersinia spp., Escherichia spp., Klebsiella spp., Bordetella spp., Neisseria spp., Aeromonas spp., Franciesella spp., Corynebacterium spp., Citrobacter spp., Chlamydia spp., Hemophilus spp., Brucella spp., Mycobacterium spp., Legionella spp., Rhodococcus spp., Pseudomonas spp., Helicobacter spp., Vibrio spp., Bacillus spp.,* and *Erysipelothrix spp.* It may be necessary to modify these bacteria to increase their invasive potential.

The particular *Yersinia* strain employed is not critical to the present invention. Examples of *Yersinia* strains which can be employed in the present invention include *Y*. *enterocolitica* (ATCC No. 9610) or *Y. pestis* (ATCC No. 19428). Attenuated *Yersinia* strains, such as Y. *enterocolitica* Ye03-R2 (al-Hendy et al. Infect. Immun., 60:870-875 (1992)) or *Y*. *enterocolitica* aroA (O'Gaora et al. Micro. Path., 9:105-116 (1990)) are preferably used in the present invention. Alternatively, new attenuated *Yersinia* strains can be constructed by introducing one or more attenuating mutations in groups (i) to (vii) as described for *Shigella spp.* above.

The particular *Escherichia* strain employed is not critical to the present invention. Examples of *Escherichia* strains which can be employed in the present invention include *E*. *coli* H10407 (Elinghorst et al. Infect. Immun., 60:2409-2417 (1992)), and *E. coli* EFC4, CFT325 and CPZ005 (Donnenberg et al. J. Infect. Dis., 169:831-838 (1994)). Attenuated *Escherichia* strains, such as the attenuated turkey pathogen *E. coli* 02 carAB mutant (Kwaga et al. Infect. Immun., 62:3766-3772 (1994)) are preferably used in the present invention. Alternatively, new attenuated *Escherichia* strains can be constructed by introducing one or more attenuating mutations in groups (i) to (vii) as described for *Shigella spp.* above.

The particular *Klebsiella* strain employed is not critical to the present invention. Examples of *Klebsiella* strains which can be employed in the present invention include *K. pneumoniae* (ATCC No. 13884). Attenuated *Klebsiella* strains are preferably used in the present invention, and can be constructed by introducing one or more attenuating mutations in groups (i) to (vii) as described for *Shigella spp.* above.

The particular *Bordetella* strain employed is not critical to the present invention. Examples of *Bordetella* strains which can be employed in the present invention include *B. bronchiseptica* (ATCC No. 19395). Attenuated *Bordetella* strains are preferably used in the present invention, and can be constructed by introducing one or more attenuating mutations in groups (i) to (vii) as described for *Shigella spp.* above.

The particular *Neisseria* strain employed is not critical to the present invention. Examples of *Neisseria* strains which can be employed in the present invention include *N. meningitidis* (ATCC No. 13077) and *N. gonorrhoeae* (ATCC No. 19424). Attenuated *Neisseria* strains, such as *N. gonorrhoeae* MS11 aro mutant (Chamberlain et al. Micro. Path., 15:51-63 (1993)) are preferably used in the present invention. Alternatively, new attenuated *Neisseria* strains can be constructed by introducing one or more attenuating mutations in groups (i) to (vii) as described for *Shigella spp.* above.

The particular *Aeromonas* strain employed is not critical to the present invention. Examples of *Aeromonas* strains which can be employed in the present invention include *A. eucrenophila* (ATCC No. 23309). Alternatively, new attenuated *Aeromonas* strains can be constructed by introducing one or more attenuating mutations in groups (i) to (vii) as described for *Shigella spp.* above.

The particular *Franciesella* strain employed is not critical to the present invention. Examples of *Franciesella* strains which can be employed in the present invention include F. *tularensis* (ATCC No. 15482). Attenuated *Franciesella* strains are preferably used in the present invention, and can be constructed by introducing one or more attenuating mutations in groups (i) to (vii) as described for *Shigella spp.* above.

The particular *Corynebacterium* strain employed is not critical to the present invention. Examples of *Corynebacterium* strains which can be employed in the present invention include C. *pseudotuberculosis* (ATCC No. 19410). Attenuated *Corynebacterium* strains are preferably used in the present invention, and can be constructed by introducing one or more attenuating mutations in groups (i) to (vii) as described for *Shigella spp.* above.

The particular *Citrobacter* strain employed is not critical to the present invention. Examples of *Citrobacter* strains which can be employed in the present invention include C. *freundii* (ATCC No. 8090). Attenuated *Citrobacter* strains are preferably used in the present invention, and can be constructed by introducing one or more attenuating mutations in groups (i) to (vii) as described for *Shigella spp.* above.

The particular *Chlamydia* strain employed is not critical to the present invention. Examples of *Chlamydia* strains which can be employed in the present invention include *C*. *pneumoniae* (ATCC No. VR1310). Attenuated *Chlamydia* strains are preferably used in the present invention, and can be constructed by introducing one or more attenuating mutations in groups (i) to (vii) as described for *Shigella spp.* above.

The particular *Hemophilus* strain employed is not critical to the present invention. Examples of *Hemophilus* strains which can be employed in the present invention include *H. sornnus* (ATCC No. 43625). Attenuated *Hemophilus* strains are preferably used in the present invention, and can be constructed by introducing one or more attenuating mutations in groups (i) to (vii) as described for *Shigella spp.* above.

The particular *Brucella* strain employed is not critical to the present invention. Examples of *Brucella* strains which can be employed in the present invention include *B. abortus* (ATCC No. 23448). Attenuated *Brucella* strains are preferably used in the present invention, and can be constructed by introducing one or more attenuating mutations in groups (i) to (vii) as described for *Shigella spp.* above.

The particular *Mycobacterium* strain employed is not critical to the present invention. Examples of *Mycobacterium* strains which can be employed in the present invention include *M. intracellulare* (ATCC No. 13950) and *M*. *tuberculosis* (ATCC No. 27294). Attenuated *Mycobacterium* strains are preferably used in the present invention, and can be constructed by introducing one or more attenuating mutations in groups (i) to (vii) as described for *Shigella spp.* above.

The particular *Legionella* strain employed is not critical to the present invention. Examples of *Legionella* strains which can be employed in the present invention include *L. pneumophila* (ATCC No. 33156). Attenuated *Legionella* strains, such as a *L. pneumophila* mip mutant (Ott, FEMS Micro. Rev., 14:161-176 (1994)) are preferably used in the present invention. Alternatively, new attenuated *Legionella* strains can be constructed by introducing one or more attenuating mutations in groups (i) to (vii) as described for *Shigella spp.* above.

The particular *Rhodococcus* strain employed is not critical to the present invention. Examples of *Rhodococcus* strains which can be employed in the present invention include *R. equi* (ATCC No. 6939). Attenuated *Rhodococcus* strains are preferably used in the present invention, and can be constructed by introducing one or more attenuating mutations in groups (i) to (vii) as described for *Shigella spp.* above.

The particular *Pseudomonas* strain employed is not critical to the present invention. Examples of *Pseudomonas* strains which can be employed in the present invention include *P*. *aeruginosa* (ATCC No. 23267). Attenuated *Pseudomonas* strains are preferably used in the present invention, and can be constructed by introducing one or more attenuating mutations in groups (i) to (vii) as described for *Shigella spp.* above.

The particular *Helicobacter* strain employed is not critical to the present invention. Examples of *Helicobacter* strains which can be employed in the present invention include *H. mustelae* (ATCC No. 43772). Attenuated *Helicobacter* strains are preferably used in the present invention, and can be constructed by introducing one or more attenuating mutations in groups (i) to (vii) as described for *Shigella spp.* above.

The particular *Salmonella* strain employed is not critical to the present invention. Examples of *Salmonella* strains which can be employed in the present invention include *Salmonella typhi* (ATCC No. 7251) and *S. typhimurium* (ATCC No. 13311). Attenuated *Salmonella* strains are preferably used in the present invention and include *S. typhi* aroC aroD (Hone et al. Vacc., 9:810-816 (1991)) and *S. typhimurium* aroA mutant (Mastroeni et al. Micro. Pathol, 13:477-491 (1992))). Alternatively, new attenuated *Salmonella* strains can be constructed by introducing one or more attenuating mutations in groups (i) to (vii) as described for *Shigella spp.* above.

The particular *Vibrio* strain employed is not critical to the present invention. Examples of *Vibrio* strains which can be employed in the present invention include *Vibrio cholerae* (ATCC No. 14035) and *Vibrio cincinnatiensis* (ATCC No. 35912). Attenuated *Vibrio* strains are preferably used in the present invention and include *V. cholerae* RSI virulence mutant (Taylor et al. J. Infect. Dis., 170:1518-1523 (1994)) and *V. cholerae* ctxA, ace, zot, cep mutant (Waldor et al. J. Infect. Dis., 170:278-283 (1994)). Alternatively, new attenuated *Vibrio* strains can be constructed by introducing one or more attenuating mutations in groups (i) to (vii) as described for *Shigella spp.* above.

The particular *Bacillus* strain employed is not critical to the present invention. Examples of *Bacillus* strains which can be employed in the present invention include *Bacillus subtilis* (ATCC No. 6051). Attenuated *Bacillus* strains are preferably used in the present invention and include *B. anthracis* mutant pX01 (Welkos et al. Micro. Pathol, 14:381-388 (1993)) and attenuated BCG strains (Stover et al. Nat., 351:456-460 (1991)). Alternatively, new attenuated *Bacillus* strains can be constructed by introducing one or more attenuating mutations in groups (i) to (vii) as described for *Shigella spp.* above.

The particular *Erysipelothrix* strain employed is not critical to the present invention. Examples of *Erysipelothrix* strains which can be employed in the present invention include *Erysipelothrix rhusiopathiae* (ATCC No. 19414) and *Erysipelothrix tonsillarum* (ATCC No. 43339). Attenuated *Erysipelothrix* strains are preferably used in the present invention and include *E. rhusiopathiae* Kg-1a and Kg-2 (Watarai et al. J. Vet. Med. Sci., 55:595-600 (1993)) and *E. rhusiopathiae* ORVAC mutant (Markowska-Daniel et al. Int. J. Med. Microb. Virol. Parisit. Infect. Dis., 277:547-553 (1992)). Alternatively, new attenuated *Erysipelothrix* strains can be constructed by introducing one or more attenuating mutations in groups (i) to (vii) as described for *Shigella spp.* above.

### 1.3. Methods for Increasing the Invasive Properties of a Bacterial Strain

Whether organisms have been traditionally described as invasive or non-invasive, these organisms can be engineered to increase their invasive properties, *e.g.,* by mimicking the invasive properties of *Shigella spp., Listeria spp., Rickettsia spp.,* or enteroinvasive *E*. *coli spp.* For example, one or more genes that enable the microorganism to access the cytoplasm of a cell, *e.g.,* a cell in the natural host of said non-invasive bacteria, can be introduced into the microorganism.

Examples of such genes referred to herein as "cytoplasm-targeting genes" include genes encoding the proteins that enable invasion by *Shigella* or the analogous invasion genes of entero-invasive *Escherichia,* or listeriolysin O of *Listeria,* as such techniques are known to result in rendering a wide array of invasive bacteria capable of invading and entering the cytoplasm of animal cells (Formal et al. Infect. Immun., 46:465 (1984); Bielecke et al. Nature, 345:175-176 (1990); Small et al. In: Microbiology-1986, pages 121-124, Levine et al. Eds., American Society for Microbiology, Washington, D.C. (1986); Zychlinsky et al. Molec. Micro., 11:619-627 (1994); Gentschev et al. (1995) Infection & Immunity 63:4202; Isberg, R. R. and S. Falkow (1985) Nature 317:262; and Isberg, R. R. et al. (1987) Cell 50:769). Methods for transferring the above cytoplasm-targeting genes into a bacterial strain are well known in the art. Another preferred gene which can be introduced into bacteria to increase their invasive character encodes the invasin protein from *Yersinia pseudotuberculosis,* (Leong et al. EMBO J., 9:1979 (1990)). Invasin can also be introduced in combination with listeriolysin, thereby further increasing the invasive character of the bacteria relative to the introduction of either of these genes. The above genes have been described for illustrative purposes; however, it will be obvious to those skilled in the art that any gene or combination of genes, from one or more sources, that participates in the delivery of a molecule, in particular an RNA or RNA-encoding DNA molecule, from a microorganism into the cytoplasm of a cell, *e.g.,* an animal cell, will suffice. Thus, such genes are not limited to bacterial genes, and include viral genes, such as influenza virus hemagglutinin HA-2 which promotes endosmolysis (Plank et al. J. Biol. Chem., 269:12918-12924 (1994)).

The above cytoplasm-targeting genes can be obtained by, *e.g.,* PCR amplification from DNA isolated from an invasive bacterium carrying the desired cytoplasm-targeting gene. Primers for PCR can be designed from the nucleotide sequences available in the art, *e.g.,* in the above-listed references and/or in GenBank, which is publicly available on the internet (www.ncbi.nlm.nih.gov/). The PCR primers can be designed to amplify a cytoplasm-targeting gene, a cytoplasm-targeting operon, a cluster of cytoplasm-targeting genes, or a regulon of cytoplasm-targeting genes. The PCR strategy employed will depend on the genetic organization of the cytoplasm-targeting gene or genes in the target invasive bacteria. The PCR primers are designed to contain a sequence that is homologous to DNA sequences at the beginning and end of the target DNA sequence. The cytoplasm-targeting genes can then be introduced into the target bacterial strain, *e.g.,* by using Hfr transfer or plasmid mobilization (Miller, A Short Course in Bacterial Genetics, Cold Spring Harbor Laboratory Press, Cold Spring Harbor, N.Y. (1992); Bothwell *et al. supra*; and Ausubel *et al. supra*), bacteriophage-mediated transduction (de Boer, *supra*; Miller, *supra;* and Ausubel *et al. supra*), chemical transformation (Bothwell *et al. supra*; Ausubel *et al. supra*), electroporation (Bothwel *et al. supra*; Ausubel *et al. supra*; and Sambrook, Molecular Cloning: A Laboratory Manual, Cold Spring Harbor Laboratory Press, Cold Spring Harbor, N.Y.) and physical transformation techniques (Johnston *et al. supra*; and Bothwell, *supra*). The cytoplasm-targeting genes can be incorporated into lysogenic bacteriophage (de Boer et al. Cell, 56:641-649 (1989)), plasmids vectors (Curtiss *et al. supra*) or spliced into the chromosome (Hone *et al. supra*) of the target strain.

In addition to genetically engineering bacteria to increase their invasive properties, as set forth above, bacteria can also be modified by linking an invasion factor to the bacteria. Accordingly, in one embodiment, a bacterium is rendered more invasive by coating the bacterium, either covalently or non-covalently, with an invasion factor, *e.g.,* the protein invasin, invasin derivatives, or a fragment thereof sufficient for invasiveness. In fact, it has been shown that non-invasive bacterial cells coated with purified invasin from *Yersinia pseudotuberculosis* or the carboxyl-terminal 192 amino acids of invasin are able to enter mammalian cells (Leong et al. (1990) EMBO J. 9:1979). Furthermore, latex beads coated with the carboxyl terminal region of invasin are efficiently internalized by mammalian cells, as are strains of *Staphylococcus aureus* coated with antibody-immobilized invasin (reviewed in Isberg and Tran van Nhieu (1994) Ann. Rev. Genet. 27:395). Alternatively, a bacterium can also be coated with an antibody, variant thereof, or fragment thereof which binds specifically to a surface molecule recognized by a bacterial entry factor. For example, it has been shown that bacteria are internalized if they are coated with a monoclonal antibody directed against an integrin molecule, *e.g.,* α5β1, known to be the surface molecule with which the bacterial invasin protein interacts (Isberg and Tran van Nhieu, *supra*). Such antibodies can be prepared according to methods known in the art. The antibodies can be tested for efficacy in mediating bacterial invasiveness by, *e.g.,* coating bacteria with the antibody, contacting the bacteria with eukaryotic cells having a surface receptor recognized by the antibody, and monitoring the presence of intracellular bacteria, according to the methods described above. Methods for linking an invasion factor to the surface of a bacterium are known in the art and include cross-linking.

### 2. Target Cells

The disclosure provides a method for delivering RNA to any type of target cell. As used herein, the term "target cell" refers to a cell which can be invaded by a bacterium, *i.e*., a cell which has the necessary surface receptor for recognition by the bacterium.

The target cells are mammalian cells.

The cells may be present in the intact animal, a primary cell culture, explant culture or a transformed cell line. The particular tissue source of the cells is not critical to the present invention.

Preferred animal cells are mammalian cells, such as human, bovine, ovine, porcine, feline, buffalo, canine, goat, equine, donkey, deer, avian, bird, chicken and primate cells. The most preferred animal cells are human cells.

In a preferred embodiment, the target cell is in a mucosal surface. Certain enteric pathogens, *e.g., E. coli, Shigella, Listeria,* and *Salmonella,* are naturally adapted for this application, as these organisms possess the ability to attach to and invade host mucosal surfaces (Kreig *et al. supra*)*.* Therefore, in the present invention, such bacteria can deliver RNA molecules or RNA-encoding DNA to cells in the host mucosal compartment.

Although certain types of bacteria may have a certain tropism, *i.e.,* preferred target cells, delivery of RNA or RNA-encoding DNA to a certain type of cell can be achieved by choosing a bacterium which has a tropism for the desired cell type or which is modified such as to be able to invade the desired cell type. Thus, *e.g.,* a bacterium could be genetically engineered to mimic mucosal tissue tropism and invasive properties, as discussed above, to thereby allow said bacteria to invade mucosal tissue, and deliver RNA or RNA-encoding DNA to cells in those sites.

Bacteria can also be targeted to other types of cells. For example, bacteria can be targeted to erythrocytes of humans and primates by modifying bacteria to express on their surface either, or both of, the *Plasmodium* vivax reticulocyte binding proteins-1 and -2, which bind specifically to erythrocytes in humans and primates (Galinski et al. Cell, 69:1213-1226 (1992)). In another embodiment, bacteria are modified to have on their surface asialoorosomucoid, which is a ligand for the asiloglycoprotein receptor on hepatocytes (Wu et al. J. Biol. Chem., 263:14621-14624 (1988)). In yet another embodiment, bacteria are coated with insulin-poly-L-lysine, which has been shown to target plasmid uptake to cells with an insulin receptor (Rosenkranz et al. Expt. Cell Res., 199:323-329 (1992)). Also within the scope of the invention are bacteria modified to have on their surface p60 of *Listeria monocytogenes,* which allows for tropism for hepatocytes (Hess et al. Infect. Immun., 63:2047-2053 (1995)), or a 60 kD surface protein from *Trypanosoma cruzi* which causes specific binding to the mammalian extra-cellular matrix by binding to heparin, heparin sulfate and collagen (Ortega-Barria et al. Cell, 67:411-421 (1991)).

Yet in another embodiment, a cell can be modified to become a target cell of a bacterium for delivery of RNA. Accordingly, a cell can be modified to express a surface antigen which is recognized by a bacterium for its entry into the cell, *i.e.,* a receptor of an invasion factor. The cell can be modified either by introducing into the cell a nucleic acid encoding a receptor of an invasion factor, such that the surface antigen is expressed in the desired conditions. Alternatively, the cell can be coated with a receptor of an invasion factor. Receptors of invasion factors include proteins belonging to the integrin receptor superfamily. A list of the type of integrin receptors recognized by various bacteria and other microorganisms can be found, *e.g.,* in Isberg and Tran Van Nhieu (1994) Ann. Rev. Genet. 27:395. Nucleotide sequences for the integrin subunits can be found, *e.g.,* in GenBank, publicly available on the internet.

As set forth above, yet other target cells include fish, avian, and reptilian cells. Examples of bacteria which are naturally invasive for fish, avian, and reptilian cells are set forth below.

Examples of bacteria which can naturally access the cytoplasm of fish cells include, but are not limited to *Aeromonas salminocida* (ATCC No. 33658) and *Aeromonas schuberii* (ATCC No. 43700). Attenuated bacteria are preferably used in the invention, and include *A. salmonicidia* vapA (Gustafson et al. J. Mol. Biol., 237:452-463 (1994)) or *A. salmonicidia* aromatic-dependent mutant (Vaughan et al. Infect. Immun., 61:2172-2181 (1993)).

Examples of bacteria which can naturally access the cytoplasm of avian cells include, but are not restricted to, *Salmonella galinarum* (ATCC No. 9184), *Salmonella enteriditis* (ATCC No. 4931) and *Salmonella typhimurium* (ATCC No. 6994). Attenuated bacteria are preferred to the invention and include attenuated *Salmonella* strains such as *S. galinarum* cya crp mutant (Curtiss *et al.* (1987) *supra)* or *S*. *enteritidis* aroA aromatic-dependent mutant CVL30 (Cooper et al. Infect. Immun., 62:4739-4746 (1994)).

Examples of bacteria which can naturally access the cytoplasm of reptilian cells include, but are not restricted to, *Salmonella typhimurium* (ATCC No. 6994). Attenuated bacteria are preferable to the invention and include, attenuated strains such as S. typhimuirum aromatic-dependent mutant (Hormaeche *et al. supra*).

Set forth below are examples of cell lines to which RNA can be delivered according to the method of this invention.

Examples of human cell lines include but are not limited to ATCC Nos. CCL 62, CCL 159, HTB 151, HTB 22, CCL 2, CRL 1634, CRL 8155, HTB 61, and HTB104.

Examples of bovine cell lines include ATCC Nos. CRL 6021, CRL 1733, CRL 6033, CRL 6023, CCL 44 and CRL 1390.

Examples of ovine cells lines include ATCC Nos. CRL 6540, CRL 6538, CRL 6548 and CRL 6546.

Examples of porcine cell lines include ATCC Nos. CL 184, CRL 6492, and CRL 1746.

Examples of feline cell lines include CRL 6077, CRL 6113, CRL 6140, CRL 6164, CCL 94, CCL 150, CRL 6075 and CRL 6123.

Examples of buffalo cell lines include CCL 40 and CRL 6072.

Examples of canine cells include ATCC Nos. CRL 6213, CCL 34, CRL 6202, CRL 6225, CRL 6215, CRL 6203 and CRL 6575.

Examples of goat derived cell lines include ATCC No. CCL 73 and ATCC No. CRL 6270.

Examples of horse derived cell lines include ATCC Nos. CCL 57 and CRL 6583.

Examples of deer cell lines include ATCC Nos. CRL 6193-6196.

Examples of primate derived cell lines include those from chimpanzee's such as ATCC Nos. CRL 6312, CRL 6304, and CRL 1868; monkey cell lines such as ATCC Nos. CRL 1576, CCL 26, and CCL 161; orangautan cell line ATCC No. CRL 1850; and gorilla cell line ATCC No. CRL 1854.

### 4. Pharmaceutical Compositions

In a preferred embodiment of the invention, the invasive bacteria containing the RNA molecules, and/or DNA encoding such, are introduced into an animal by intravenous, intramuscular, intradermal, intraperitoneally, peroral, intranasal, intraocular, intrarectal, intravaginal, intraosseous, oral, immersion and intraurethral inoculation routes.

The amount of the live invasive bacteria of the present invention to be administered to a subject will vary depending on the species of the subject, as well as the disease or condition that is being treated. Generally, the dosage employed will be about 10³ to 10¹¹ viable organisms, preferably about 10⁵ to 10⁹ viable organisms per subject.

The invasive bacteria of the present invention are generally administered along with a pharmaceutically acceptable carrier and/or diluent. The particular pharmaceutically acceptable carrier an/or diluent employed is not critical to the present invention. Examples of diluents include a phosphate buffered saline, buffer for buffering against gastric acid in the stomach, such as citrate buffer (pH 7.0) containing sucrose, bicarbonate buffer (pH 7.0) alone (Levine et al. J. Clin. Invest., 79:888-902 (1987); and Black et al J. Infect. Dis., 155:1260-1265 (1987)), or bicarbonate buffer (pH 7.0) containing ascorbic acid, lactose, and optionally aspartame (Levine et al. Lancet, II:467-470 (1988)). Examples of carriers include proteins, *e.g.,* as found in skim milk, sugars, *e.g.,* sucrose, or polyvinylpyrrolidone. Typically these carriers would be used at a concentration of about 0.1-30% (w/v) but preferably at a range of 1-10% (w/v).

Set forth below are other pharmaceutically acceptable carriers or diluents which may be used for delivery specific routes. Any such carrier or diluent can be used for administration of the bacteria of the invention, so long as the bacteria are still capable of invading a target cell. *In vitro* or *in vivo* tests for invasiveness can be performed to determine appropriate diluents and carriers. The compositions of the invention can be formulated for a variety of types of administration, including systemic and topical or localized administration. Lyophilized forms are also included, so long as the bacteria are invasive upon contact with a target cell or upon administration to the subject. Techniques and formulations generally may be found in Remmington's Pharmaceutical Sciences, Meade Publishing Co., Easton, Pa. For systemic administration, injection is preferred, including intramuscular, intravenous, intraperitoneal, and subcutaneous. For injection, the composition, *e.g.,* bacteria, of the invention can be formulated in liquid solutions, preferably in physiologically compatible buffers such as Hank's solution or Ringer's solution.

For oral administration, the pharmaceutical compositions may take the form of, for example, tablets or capsules prepared by conventional means with pharmaceutically acceptable excipients such as binding agents (*e.g*., pregelatinized maize starch, polyvinylpyrrolidone or hydroxypropyl methylcellulose); fillers (*e.g.,* lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g.,* methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the compositions may take the form of tablets or lozenges formulated in conventional manner.

For administration by inhalation, the pharmaceutical compositions for use according to the present invention are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g*. gelatin for use in an inhaler or insufflator may be formulated containing a powder mix of the composition, *e.g.,* bacteria, and a suitable powder base such as lactose or starch.

The pharmaceutical compositions may be formulated for parenteral administration by injection, *e.g*., by bolus injection or continuous infusion. Formulations for injection may be presented in unit dosage form, *e.g.*, in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

The pharmaceutical compositions may also be formulated in rectal, intravaginal or intraurethral compositions such as suppositories or retention enemas, *e.g*., containing conventional suppository bases such as cocoa butter or other glycerides.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the bacteria of the invention are formulated into ointments, salves, gels, or creams as generally known in the art, so long as the bacteria are still invasive upon contact with a target cell.

The compositions may, if desired, be presented in a pack or dispenser device and/or a kit which may contain one or more unit dosage forms containing the active ingredient. The pack may for example comprise metal or plastic foil, such as a blister pack. The pack or dispenser device may be accompanied by instructions for administration.

The invasive bacteria containing the RNA or RNA-encoding DNA to be introduced can be used to infect animal cells that are cultured *in vitro,* such as cells obtained from a subject. These *in vitro*-infected cells can then be introduced into animals, *e.g*., the subject from which the cells were obtained initially, intravenously, intramuscularly, intradermally, or intraperitoneally, or by any inoculation route that allows the cells to enter the host tissue. When delivering RNA to individual cells, the dosage of viable organisms to be administered will be at a multiplicity of infection ranging from about 0.1 to 10⁶, preferably about 10² to 10⁴ bacteria per cell.

In yet another embodiment of the present invention, bacteria can also deliver RNA molecules encoding proteins to cells, *e.g.,* animal cells, from which the proteins can later be harvested or purified. For example, a protein can be produced in a tissue culture cell.

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

The present invention is further illustrated by the following examples which should not be construed as limiting in any way. The contents of all cited references including literature references, issued patents, published patent applications as cited throughout this application are hereby expressly incorporated by reference. The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Pat. No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R.I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### EXAMPLES

### Methods

### siRNA-generating plasmid construction:

Oligonucleotides were obtained at 0.2 µmol from QIAGEN with PAGE purification. After annealing, oligonucleotides were inserted into the BamHI and HindIII binding sites within pSilencer 2.0-U6 (Ambion, Inc.) according to the manufacturer's instructions.

The following sequences were used:
The *k-Ras-*1 and -2 64-mers target the nucleotides encoding for amino acids 9-15 of *k-Ras* protein which spans the specific mutation of V12.
   *k-Ras*-1 (64-mer): 
5'gATCCCgTTggAgCTgTTggCgTAgTTCAAgAgACTACgCCAACAgCTCCAACTTTTTT
ggAAA3' (SEQ ID NO:95)
   *k-Ras*-2 (64-mer):

5’AgCTTTTCCAAAAAAgTTggAgCTgTTggCgTAgTCTCTTgAACTACgCCAAC
AgCTCCAACgg3’ (SEQ ID NO: 96)

The β-*Catenin*-1 and -2 64-mers target the nucleotides encoding for amino acids 79-85 within the catenin protein
β*-Catenin*-1 (64mer): 
5'gATCCCAgCTgATATTgATggACAgTTCAAgAgACTgTCCATCAATATCAgCT
TTTTTggAAA3' (SEQ ID NO: 97)
β*-Catenin*-2 (64mer):

5'AgCTTTTCCAAAAAAAgCTgATATTgATggACAgTCTCTTgAACTgTCCATC
AATATCAgCTgg3' (SEQ ID NO:98)

The EGFP-1 and -2 64-mers target the nucleotides encoding for amino acids 22-28 of EGFP.
EGFP-1 (64-mer): 
5'gATCCCgACgTAAACggCCACAAgTTTCAAgAgAACTTgTggCCgTTTACgTC
TTTTTTggAAA3' (SEQ ID NO:99)
EGFP-2 (64-mer):

5'AgCTTTTCCAAAAAAgACgTAAACggCCACAAgTTCTCTTgAAACTTgTggC
CgTTTACgTCgg3' (SEQ ID NO:100)

### Transkingdom RNA Interference Plasmid Construction:

The engineered plasmid pT7RNAi-Hly-Inv, TRIP was constructed from pGB2Ωinv-hly (Milligan et al., Nucleic Acids Res. 15, 8783 (1987)) and pBlueScript II KS(+). Oligonucleotides containing multiple cloning site (MCS), T7 promoter, enhancer and terminator (synthesized from Qiagen) were ligated into blunted BssHII sites of KSII(+), and β*-catenin* hairpin oligos were inserted into BamHI and SalI sites of MCS to generate plasmid pT7RNAi. PstI fragments containing the inv locus of pGB2Ωinv-hly were inserted into PstI site of KSII(+). Using pGB2Ωinv-hly as template, HlyA gene was amplified by PCR (Pfx DNA polymerase, Invitrogen Inc.) with primers, hly-1: 5'-CCCTCCTTTGATTAGTATATTCCTATCTTA-3' (SEQ ID NO:101)and hly-2: 5'-AAGCTTTTAAATCAGCAGGGGTCTTTTTGG-3' (SEQ ID NO:102), and were cloned into EcoRV site of KSII(+)/Inv. Hly-Inv fragment was excised with BamHI and SalI. After blunting, it was ligated into EcoRV site incorporated within T7 terminator of pT7RNAi

### Bacteria:

The auxotrophic *Salmonella typhimurium* aroA 7207 (*S*. *typhimurium* 2337-65 derivative hisG46, DEL407[aroA544::Tn10(Tc-s)] used as the plasmid carrier in this study was kindly provided by Prof. BAD Stocker, Stanford University, CA. *Escherichia coli* XL-1 Blue was used to maintain the plasmids (Strategene).

Transformation of SL 7207 was achieved using an adapted electroporation protocol (1). Competent SL7207 and 1 µg plasmid were incubated on ice in a chilled 0.2 cm electroporation cuvette for 5 min. A 2.5 kV, 25 µF, 200 Ω impulse was applied using a BioRad Genepulser. 1 mL of prewarmed SOC medium was added and bacteria were allowed to recover for 1 hr at 37 °C with 225 RPM shaking before plating on selective agar plates. Presence of the plasmids was confirmed using minipreparation after alcalinic lysis and separation on 0.7% agarose gel.

For *in vitro* experiments, SL 7202 were grown overnight at 37 °C in Luria Broth (LB) supplemented with 100 µg/mL Ampicillin (for SL-siRAS, SL-siGFP and SL-siCAT) without shaking. The next morning, bacteria were grown in fresh medium after 1% inoculation from the overnight culture until reaching an OD₆₀₀ of 0.4-0.6. Bacteria were centrifuged (3500 RPM, 4 °C) washed once in phosphate-buffered saline (PBS) and resuspended in PBS at the desired concentrations. For all determinations of bacterial number and concentration, the bacterial density was measured spectrometrically and calculated according to the formula c = OD₆₀₀ * 8x10⁸/mL.

For animal experiments, SL 7207 were grown in Brain Heart Infusion Broth (Sigma) in a stable culture overnight supplemented with the appropriate antibiotics where required. Bacteria were washed and resuspended in PBS at a concentration of 2.5x10¹⁰/mL. Serial dilutions were done and plated on selective agar at several times during the experiment to verify the actual number of bacteria administered.

Plasmids were also transformed into BL21DE3 strain (Gene Therapy Systems) according to the manufacturer instructions. Bacteria were grown at 37°C in Brain-Heart-Infusion-broth with addition of 100 µg/ml Ampicillin. Bacteria numbers were calculated using OD₆₀₀ measurement. For cell infection, overnight cultures were inoculated into fresh medium for another 2 h growth.

### Cell Culture:

A human colon cancer cell line (SW 480) was used herein. It carries a mutation of APC protein resulting in increased basal levels of β*-catenin.* A stably GFP-expressing cell line derived from yolk sac epithelium, CRL 2583 (ATCC, Rockville, MD) was used for GFP-knockdown experiments. CRL 2583 was maintained in 200 µg/mL G418 until 30 min before bacterial infection. SW 480 were grown in RPMI-1640 supplemented with 10% fetal bovine serum. CRL 2583 were grown in high glucose, high NaHCO₃ DMEM supplemented with 15% FBS as recommended by the supplier. All growth media were routinely supplemented with antibiotics: 100 U/ml penicillin G, 10µg/ml streptomycin, 2.5 µg/ml amphotericin (all media and additives purchased from Sigma, St.Louis).

For direct transfection of plasmids, 500,000 cells were seeded into 6 cm petri dishes and allowed to grow overnight before they were transfected using a standard CaP-coprecipitation protocol.

Briefly, 15 µg plasmid-DNA are mixed in 500 µL reaction mix (2x HEPES buffer, 60 µL CaP) and dropped to the cells in fresh medium without FBS. Precipitation was allowed to continue for 9 hrs before precipitates were washed away. Cells were harvested at different time points (36, 48, 60, 72, and 96 hrs).

For standard bacterial infection assays, 500,000 cells were seeded into 6 cm petri dishes and were allowed to attach overnight. 30 min prior to addition of the bacteria, the growth medium was replaced with fresh medium without antibiotics and fetal bovine serum. SL 7207-siRAS, -siCAT, -siGFP were added in 500 µL PBS to reach the designated multiplicity of infection (MOI) of 100, 500 or 1000 and infection was carried out in a standard incubator with 37 °C, 5% CO₂. By the end of the indicated infection period, plates were washed once with 4 ml of serum-free RPMI medium and 3 times with 4 ml PBS, then 5 ml of fresh complete RPMI medium containing 100 µg/mL of ampicillin and 150 µg/mL of gentamycin were added. Twenty-four hours later, tetracycline was added to final concentration of 15 µg/mL. At indicated different time points (24-96h) after bacterial invasion, cells were harvested for western blot or flow cytometry.

For staining of intracellular bacteria, cells were grown on Lab-Tek II Chamber Slides (Nalgene Nunc, USA). After bacterial invasion as described above, cells were washed with PBS and fixed in 1% paraformaldeyde for 10 min. Acridin Orange (Sigma) solution (0.01 %) was added for 45 sec, then washed with PBS. Crystal Violet stain (Sigma) was applied for 45 sec, then washed with PBS. Coverslips were mounted using PERMOUNT™ mounting medium and invasion was assessed using confocal microscopy.

### MTT Assay:

After treatment with SL7207-siRAS and/or SL7207-siCAT, cells were trypsinized (24h or 48h later), diluted and seeded into 96-well plate at a concentration of 5000 cells/well. Cells were then allowed to grow for up to 4 days. At the desired incubation time point, medium was removed and 100µl of MTT solution (5 mg/mL) was added to each well. After an incubation period of 4 h, MTT solution was drained away and cells were lysed by adding 100µL of solubilization reagent (Isopropanol:1N HCl:10% SDS 43:2:5) to each well. The resulting signal of the dark blue formazan-product was photometrically determined at 570 nm wavelength. The amount of color formation is dependent on the number of surviving cells per well.

### Colony Formation Test:

After treatment with SL7207-siRAS and/or SL7207-siCAT, cells were trypsinized (24 h post-transfection), diluted and seeded into 6-well MTP at a concentration of 750 cells/well. Cells were kept growing for two more weeks to let them form visible colonies. Two weeks later, medium was removed and 1ml of Giemsa stain (7.415g/L) were added to each well. After 10-min incubation at 37 °C, Giemsa stain was drained away and cells washed with PBS. Groups of more than eight cells were counted as positive colonies.

### Western Blot:

Cells were washed with chilled PBS, scraped off and lysed in lysis buffer (50mM HEPES pH 7.5,150 mM NaCl, 1 mM EDTA, 2.5 mM EGTA, 1% NP-40, 1mM DTT) containing 0.1 % protease inhibitor mix (Sigma). 20 µg of protein were separated using 11% SDS-Page Gel and transferred to a 0.4 µm PVDF membrane (Schleicher and Schuell). The membrane was blocked using 5% milk and incubated for 1 hr with specific antibodies at the indicated concentrations: Living Colors® antibody (Clontech)-1:500, β*-catenin* antibody (Santa Cruz)-1:500, *k-Ras* antibody (Santa Cruz)-1:300 and β-actin (Santa Cruz) 1:500. Each was followed by incubation with horseradish-peroxidase conjugated anti-rabbit or anti-goat secondary antibodies (Santa Cruz)-1:1000-1:2000. Bands were detected using ECL® chemoluminescence detection (Amersham).

### Flow Cytometry:

For flow cytometry, cells were trypsinized for 3 min, resuspended in fresh medium and washed in PBS. After centrifugation, cells were fixed for 10 min in 1% paraformaldehyde/PBS at 4 °C. Flow cytometry was performed using FACScan (Becton Dickinson), data analysis was done using CellQuest ® software.

### Animal Techniques:

Six to eight week old female GFP+ transgenic mice (CgTg5Nagy) were obtained from Jackson laboratories. They were housed in the BIDMC animal research facility with *ad libitum* access to standard rodent diet and drinking water. Treatment was initiated at ten weeks of age. For the iv treatment protocol, four doses of 10⁶ cfu SL-siRAS or SL-siGFP dissolved in 50 µL PBS were injected into the tail vein on alternating days. Mice were weighed daily and monitored for signs of disease.

Mice were sacrificed one day after the final treatment at which time tissue samples were taken for histochemistry and flow cytometric analysis. Tissues were paraffin embedded and sectioned in 6 µm steps for histochemisty and fluorescence microscopy. For flow cytometry, hepatocyte and splenocyte suspensions were prepared through the use of cell strainers (Falcon). Organ suspensions were fixed in 4% formalin and flow cytometry was performed using FACScan (Becton Dickinson), data analysis was done using CellQuest® software.

For the Xenograft cancer model, female BALB/c nude mice (Charles River Laboratories) were randomized into two groups (n=6). Three weeks before treatment, 1x10⁷ of SW480 cells were implanted subcutaneously. Treatments were initiated when the tumors reached about 10 mm in diameter. The treatment group was injected through tail vein with 1x10⁸ cfu *o*f *E. coli* expressing shRNA against *β-catenin* in PBS. The control group was similarly treated except that the *E. coli* contains the TRIP vector without shRNA insert. The treatment was carried out every 5 days for a total of three treatments. Mice were sacrificed 5 days after the last treatment. Tissues were frozen and fixed for analysis of *β-catenin* mRNA level by real-time PCR and *β*-*catenin* protein level by immunohistochemistry.

For *in vivo* silencing experiments, female C57/BL6 mice (Charles River Laboratories) were randomly divided into two groups. The treatment group was administered orally with 5x10¹⁰ cfu *E. coli* expressing shRNA against *β*-*catenin* in 200µL phosphate-buffered saline (PBS). The control group was similarly treated except that the *E. coli* contains the TRIP vector without the shRNA insert. Two independent experiments were performed with 6 and 5 mice per group used, respectively. The treatment was carried out daily for 5 days per week for a total of 4 weeks. Mice were sacrificed 2 days after the last treatment, and tissues were paraffin-embedded.

### Immunohistochemistry

Immunostaining was performed on 6 µm tissue sections using Vectastain Elite ABC avidin-biotin staining kit (Vector laboratories, Burlingame, CA) according to the instructions by the manufacturer. Slides were deparaffinized and rehydrated using a standard protocol. For antigen retrieval, slides were heated by microwave in 5% urea for 5 min. Unspecific binding sites were blocked with 1 % bovine serum albumin for 10 min and endogenous peroxidase activity was suppressed by treatment with 3% H₂O₂ in methanol for 10 min. Sections were exposed to primary antibody LIVING COLORS™ rabbit polyclonal antibody (Clontech) at 1:500 dilution overnight at 4 °C. The chromogen used was 3,3'Diamino-enzidine (DAB) (Vector), counterstaining was done with hematoxyline.

### Interferon response detection:

SW480 cells were treated for 2 h with *E. coli* transformed with the TRIP encoding shRNA against human *β*-*catenin* or mutant *k-Ras* at MOI of 1:1000. Untreated cells were used as control. Cells were harvested at 24, 48 and 72 h. The expression levels of OAS1, OAS2, MX1, ISGF3γ and IFITM1 genes were determined by RT-PCR using the Interferon Response Detection Kit (SBI System Biosciences, CA).

### Example 1: Knock down of green fluorescent protein using bacteria mediated gene silencing in vitro and in vivo.

In the following experiments, an attenuated strain of *Salmonella typhimurium* (SL 7207, obtained from BAD Stocker, Stanford University) was used. To prove that the concept is useful as a general approach, we did confirmation experiments also with another attenuated strain of *Salmonella typhimurium* (VNP 70009, obtained from VION Pharmaceuticals, New Haven) and an invasive and attenuated strain of *E. coli* (BM 2710, obtained from P.Courvalin, Institut Pasteur, Paris).

Silencing plasmids were designed based on a commercially available plasmid (pSilencer, Ambion) to knock down the target genes GFP, *β-catenin* and oncogenic *k-Ras* (V12G). These plasmids were transformed into SL 7207 by electroporation and positive clones were verified by growth on selective agar and DNA preparation.

For *in vitro* use, knockdown of GFP expression was demonstrated using the stable GFP+ cell line CRL 2598 (ATCC, Rockland, Va). Knockdown of oncogenic *k-Ras* (V12G) *and β*-*catenin* was demonstrated using the colon cancer cell line SW 480 and the pancreatic cancer cell line CAPAN-1.

A system of bacterial delivery using an invasive bacterial strain, *S. typhimurium,* was developed with a commercially available eukaryotic transcription plasmid, pSilencer (Ambion). The *S. typhimurium* strain SL 7207 (kindly provided by B.Stocker, Stanford University) is attenuated through an auxotrophy in the synthesis pathway for aromatic amino acids, and dies quickly after invasion into a target cell due to lack of nutrients. This strain has been used successfully for delivery of DNA *in vitro* and in mouse models, mainly with the purpose of DNA vaccination.

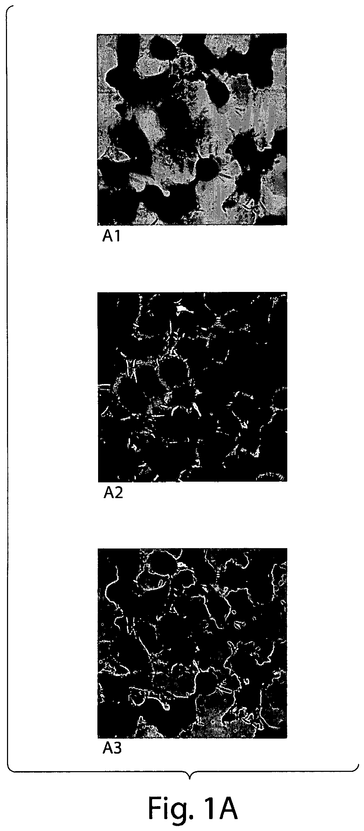

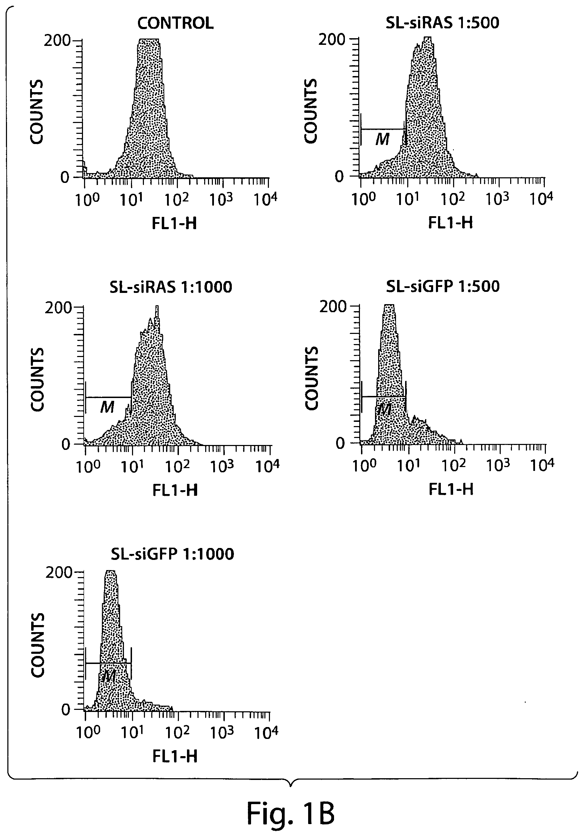

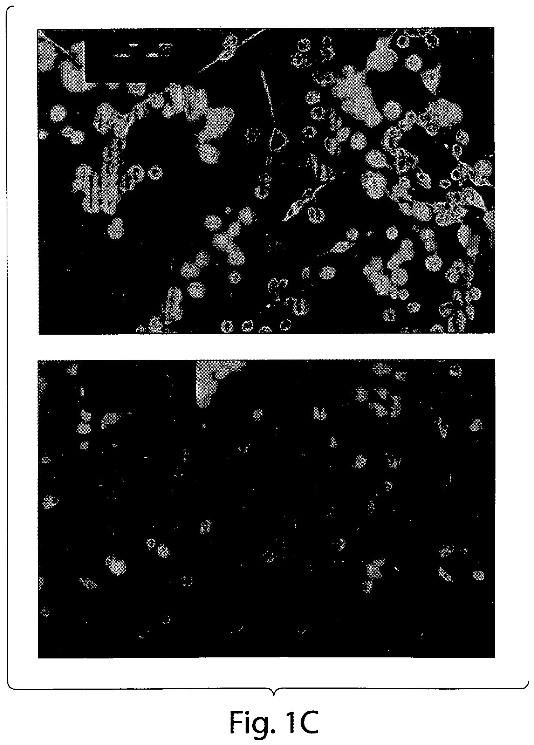

To verify bacterial entry into epithelial cells, an invasion assay was performed. SW 480 cells were infected for 2 hrs with SL-siRAS followed by 2 hrs of treatment with gentamycin. Acridin orange/crystal violet staining revealed good invasion efficiency. 90% of the SW 480 cells harbored viable SL 7207 bacteria. The average number of intracellular bacteria was 6 (range, 2-8) (Fig 1). (Micrograph A1 is the transmission image. Micrograph A2 is the fluorescent image. Micrograph A3 is the merged image.) The number of viable intracellular bacteria reduced quickly over time. After 24 hrs and 48 hrs, only 10% and 3% of cells were found to still contain bacteria.

In the next experiment, The effective reduction of GFP expression in the GFP+ cell line was demonstrated. Successful knockdown of oncogenes *k-Ras* and *β-catenin* was confirmed using Western blot and RT-PCR. Oncogene knockdown resulted in growth retardation and decreased tumor formation in a xenograft animal model.

Cells stably expressing GFP (CRL 2583) were infected with SL7207 carrying pSilencer2.0 including a sequence to silence GFP mRNA (SL-siGFP). (*See* above). After 48hrs, cells treated with SL-siGFP showed a marked decrease in GFP expression as compared to cells treated with SL-siRAS and untreated control (Figs 1B and 1C). Treatment with SL-siGFP led to loss of GFP signal in a manner dependent on the multiplicity of infection (MOI) applied. In untreated cells, only 4.3% display low or absent fluorescence. In SL-siGFP treated cells, this fraction increased to 78.1% (treated with MOI 1:500) and 92.3% (MOI 1:1000). Control treatment with SL-siRAS lead to a slight loss of fluorescence (7.5% at MOI 1:500 and 8.4% at MOI 1:1000). This is also shown in the fluorescence microscope photograph in Fig. 1C. The top micrograph is (200x) of SL-siRAS and the bottom of SL-siGFP (below) treated CRL 2583 cells. This finding was confirmed using flow cytometry.

In a series of animal experiments with stably GFP-expressing mice, we were able to demonstrate knockdown of GFP expression in the liver and in the colon (in both organs approx 50% reduction of GFP expression) after oral and intravenous application of SL 7207 carrying the GFP-silencing plasmid.

In animal experiments, *S. typhimurium* was used to achieve gene silencing in a transgenic mouse model (GFP+). Using this method, silencing of the transgene in the animal experiment is demonstrated at mRNA level as well as on protein levels and tissue sections of various organs (liver, gastrointestinal tract) with limited toxicity.

### Example 2. Knock down of k-Ras and β-catenin using bacteria mediated gene silencing.

Next, BMGS was applied to knock down a specific disease-related gene. The specific oncogenic point mutation in the *k-Ras* gene, *k-Ras*^{V12G}, which is present in the human colon carcinoma cell line, SW 480 was targeted.

After construction of the silencing plasmids and before they were electroporated into the attenuated SL7207, their activity was tested by transfecting them into SW 480 cells using CaP coprecipitation.

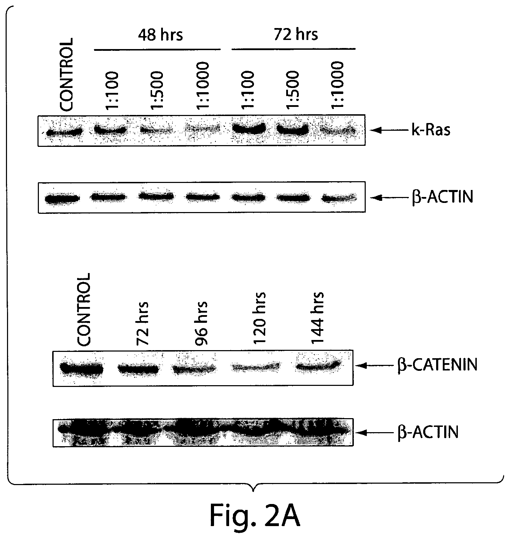

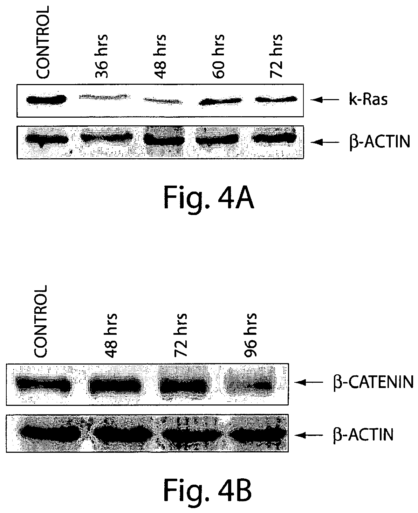

Western blot (Fig. 4A) shows efficient knockdown of *k-Ras* using the pSilencer-kras (V12G) insert at 36h and 48h posttransfection. At later time points, the protein expression recovers, which is due to outgrowth of transfected clones which have a growth disadvantage versus non transfected clones in which the oncogenic *k-Ras* would still drive replication. When BMGS with SL7207 as a carrier was used to mediate the knockdown, *k-Ras* levels were decreased at MOI of 1:500 and 1:1000. Using BMGS, knockdown of the *k-Ras* protein was observed with similar efficiency compared to direct transfection of the silencer plasmid using calcium-phosphate coprecipitation, although the onset of knockdown was slightly delayed by 12 hrs. (Fig 4A). With an MOI of 1:1000, the result can be observed for a longer time (up to 72 hrs) (Fig 2A).

The Western blot for *β*-*catenin* (Fig 4B) shows delayed knockdown after transfection, with a maximum effect seen at 96 hrs post transfection. It is assumed that this delay is caused by the survival time of SL 7207 intracellularly before the plasmid is liberated (Fig 2A).

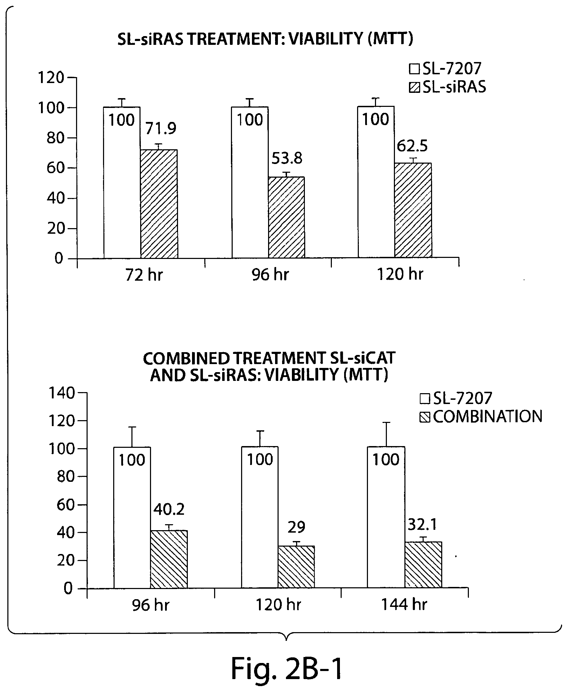

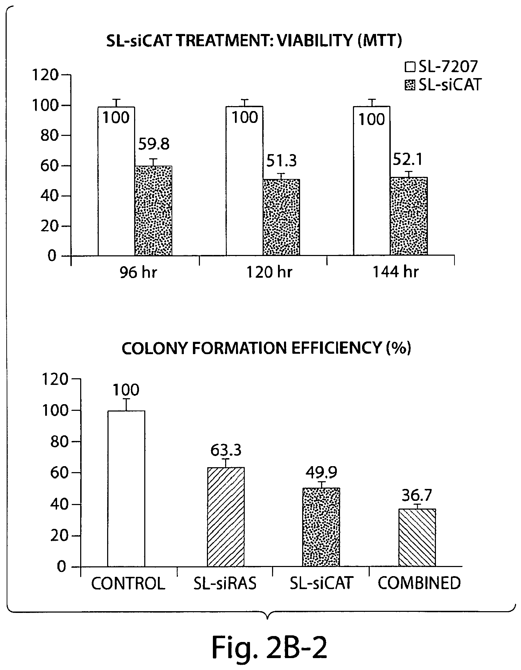

After treatment with SL-siRAS and resulting knockdown of the oncogenic *k-Ras* (V12G), SW 480 cells displayed significantly reduced viability and colony formation ability (Fig 2B). Cells were coincubated with equal amounts (2.5 x 10⁸) of SL-siRAS and SL-siCAT bacteria. Control cells were treated with untransformed SL 207. 48 hrs after transfection, cells were seeded in 96 well plates for MTT test and 6 well plates for colony formation assays. At 120 hrs after treatment, viability, as assessed by MTT assay, was reduced to 62.5% after SL-siRAS treatment and 51% after SL-siCAT treatment. Combined treatment further reduced viability to 29% of control treated cells. SL-siRAS treatment and SL-siCAT treatment reduced the ability of SW 480 to form colonies by 37.7 % and 50%, respectively. Combined treatment lead to 63.3% reduction.

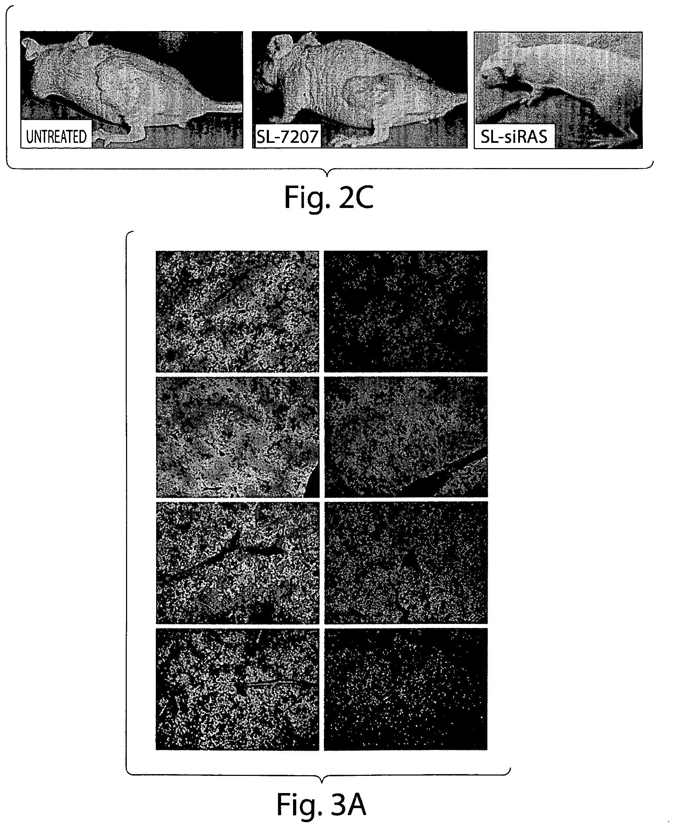

Further, treatment with SL-RAS completely inhibited the tumor formation ability of SW 480 cells when injected subcutaneously into nude mice, while treatment with empty SL 7207 did not influence their ability to form tumors (Fig 2C). SW 480 cells (untreated, treated with SL 7207 or SL-siRAS) were subcutaneously injected into nude mice (4x10⁶ cells, n = 4 animals per group). Pretreatment with SL-siRAS completely abolished the ability to form tumors (no tumors visible in any of the four animals, day 40) (Fig. 2C).

To test whether this approach can be employed universally, another cancer-related gene, *β-catenin* was targeted (Fig. 2A). Basal levels of *β**-**catenin* are high in SW 480 cells, due to a mutated APC-gene, but can be reduced through treatment with hairpin siRNA after pSilencer(siCAT) transfection (Fig 2A). After treatment with SL 7207 carrying pSilencer with the *β-catenin* construct (SL-siCAT), significant knockdown of *β-catenin* expression was achieved which resulted in decreased viability and colony formation ability (Fig2A). *β-catenin* was knocked down from 96hr, but recovered from 144hr.

### Example 3: In vivo bacterial mediated gene silencing.

The method of bacterial mediation of RNAi offers the possibility of selectively targeting more than one gene at a time which might allow for increased efficiency for future applications, *e.g.* anticancer treatment through interference with multiple oncogenic pathways. To test the feasibility of such an approach, both the mutated *k-Ras* oncogene and *β-catenin* were targeted simultaneously. After simultaneous treatment with SL-siRAS and SL-siCAT, knockdown of both genes was observed at the protein level and resulted in further decreased viability and colony formation ability (Fig 2). These findings demonstrate that the proposed concept of bacterial mediated gene silencing can be successfully used *in vitro* for different target genes and in different cell lines.

A mouse model was chosen to test whether this approach can be used to silence target genes *in vivo.* CgTg5-Nagy mice express high levels of GFP in all tissues. 14 mice were randomly assigned to receive four doses of 10⁶ cfu of either SL-siGFP or SL-siRAS i.v. on alternating days (seven animals per group). This treatment was well tolerated with no weight loss or clinically apparent signs of disease. All mice were sacrificed one day after the last treatment.

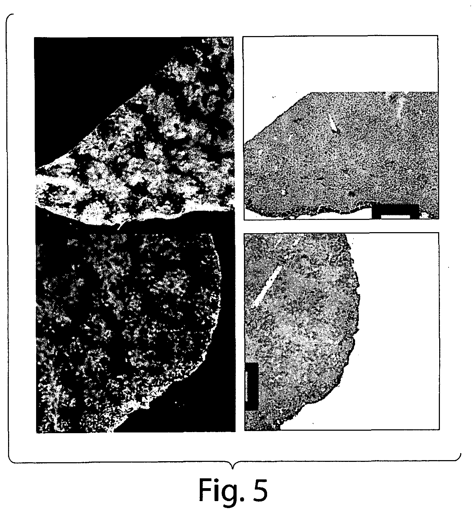

Liver tissue slides were assessed by fluorescence microscopy and immunohistochemistry with GFP antibody. (Fig 3A). Intravenous treatment with SL-siGFP led to decrease of fluorescence in the liver sections of the treated animals compared with SL-siRAS treated control animals. Histochemistry staining, with anti-GFP antibody, verified that changes in fluorescence were caused by a reduction in GFP and not caused by changes in background fluorescence levels. (Fig. 5) To verify that reductions in fluorescence in the liver sections of treated mice are really caused by changes in GFP expression levels and not due to changes in background fluorescence, tissue slides were stained with GFP specific antibody.

Immunohistochemical staining patterns correlate well with fluorescence microscopy images and confirm that changes in fluorescence are caused by decreased GFP expression. Fluorescence microscopy (50x) and corresponding immunohistochemistry image (50x) of liver section from control (top row) and iv treated (lower row) animal.

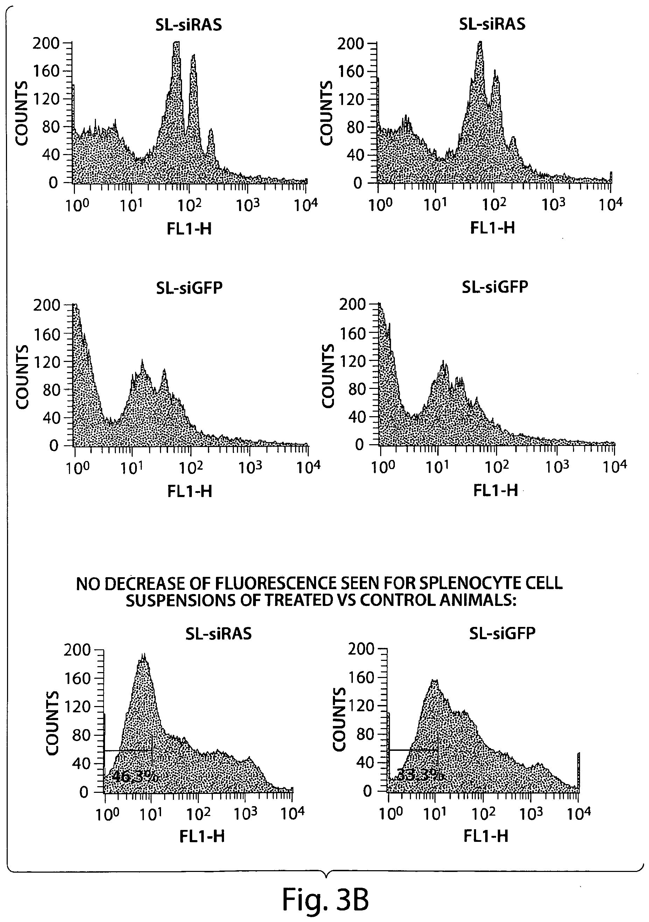

Staining patterns correlated well with fluorescence images. Subsequent image analysis revealed reductions in the number of GFP expressing cells between 9-25% after SL-siGFP treatment. These findings were confirmed by flow cytometric analysis of single cell suspensions of hepatocytes which showed a significant decrease in the number of GFP-positive hepatocytes in SL-siGFP treated vs SL-siRAS treated animals (Fig 3B). Flow cytometry measurements of hepatocyte and splenocyte suspensions were performed. After intravenous treatment with SL-siGFP, the number of GFP+ hepatocytes was significantly reduced compared to control treated (SL-siRAS) animals (SL-siRAS: 50.0% [45.4-53.2%], SL-siGFP: 39.9% [26.1-53.2%], p<0.05).

These results indicate that significant gene silencing can be achieved *in vivo* using this approach. Using iv application of attenuated *S. typhimurium* we were able to extend the *in vitro* findings into a mouse model and achieve significant gene silencing in the liver. Other organs might become accessible through use of different invasive bacterial strains or different routes of application. Professional phagocytes will be a promising target for bacteria-mediated gene silencing, as transfection efficiencies have been reported to be higher for these cells compared to cells of epithelial lineage.

### Example 4: Transkingdom RNA Interference

The use of bacteria-mediated RNAi in higher organisms holds the potential for functional genomics in mammalian system, as previously demonstrated in *C*. *elegans,* and for other *in vivo* applications of RNAi. To investigate this possibility, the bacterial plasmid pT7RNAi-Hly-Inv, termed TRIP (transkingdom RNA interference plasmid) was constructed (Fig. 6A). In this novel plasmid construct, the expression of shRNA was directed under the bacteriophage T7 promoter (Milligan and Uhlenbeck, Methods Enzymol. 180, 51-62 (1989) and Milligan et al., Nucleic Acids Res. 15, 8783-8798 (1987), rather than a mammalian promoter or enhancer. The shRNA can only be produced by the bacterial system. The TRIP vector contains the *Inv* locus that encodes invasion (Isberg et al., Cell 50, 769-778 (1987)), which permits the non-invasive *E. coli* to enter β1-integrin-positive mammalian cells (Young et al., J. Cell Biol. 116, 197-207 (1992)). The TRIP vector also contains the *Hly A* gene that encodes listeriolysin O to permit genetic materials to escape from entry vesicles (Mathew et al., Gene Ther. 10, 1105-1115 (2003) and Grillot-Courvalin et al., Nat. Biotechnol. 16, 862-866 (1998)). TRIP constructs were introduced into a competent strain of non-pathogenic *E*. *coli,* BL21DE3, which contains T7 RNA polymerase to express shRNA. A TRIP against the cancer gene *β*-*catenin* was constructed as an example. Activation of the *β-catenin* pathway from over-expression or oncogenic mutation of *β-catenin* is responsible for the initiation of the vast majority of colon cancers and is involved in a variety of other cancer types (Kim et al., Oncogene 24, 597-604 (2005)). Despite the potential of *β*-*catenin* as a cancer therapeutic target, the *β*-catenin pathway has been recalcitrant to inhibition by small molecules. *β-catenin* is a preferred choice in proof of concept experiments for testing the potency of new a RNAi approach because it is commonly stabilized in cancer cells. TRIP can be modified to enable bacteria to express interfering RNA against various genes of interest.

To determine if gene silencing can be achieved through this transkingdom system, human colon cancer cells (SW 480) were co-cultured *in vitro* with *E. coli* for 2 h (Fig. 6B and 6D) or different time (Fig. 6C), then treated with antibiotics to remove extracellular bacteria. Cells were further cultured for 48 h before harvest for analysis of gene silencing. As shown in Fig. 6B-6D, *β*-*catenin* was potently and specifically silenced at protein and mRNA level, while *β-actin, k-Ras,* and glyceraldehyde-3-phosphate dehydrogenase (GAPDH) were not affected. To further test the specificity of the transkingdom RNAi, *E. coli* containing the TRIP against mutant *k-Ras* (GGT→GTT at codon 12) silenced *k-Ras* expression in SW 480 cells with the same codon 12 mutation, but not in DLD1 cells with mutation in a different codon of *k-Ras* (GGC→GAC at codon 13, Fig. 6E). As an shRNA control, *E. coli* containing the TRIP against wild type *k-Ras* exerted no gene-silencing effect on mutated *k-Ras* in SW 480 cells (Fig. 6F). These results show that the transkingdom RNA interference is highly gene-specific, sufficient to discriminate a point mutation.

To investigate the variables that affect the potency of gene silencing by the transkingdom system, cells were incubated for 2 h with the *E. coli* at different multiplicity of infection (MOI). As shown in Fig. 6B, the potency of gene silencing was dependent on MOI, with near complete gene silencing at a MOI of 1:1,000. To determine the effect of co-culture time on gene silencing, cells were incubated with the *E. coli* at a MOI of 1:500 for different times. As shown in Fig. 6C, gene-silencing potency increased with incubation time up to 2 h. The dependency of gene silencing on MOI and co-culture time provides controllable flexibility for gene silencing in various applications.

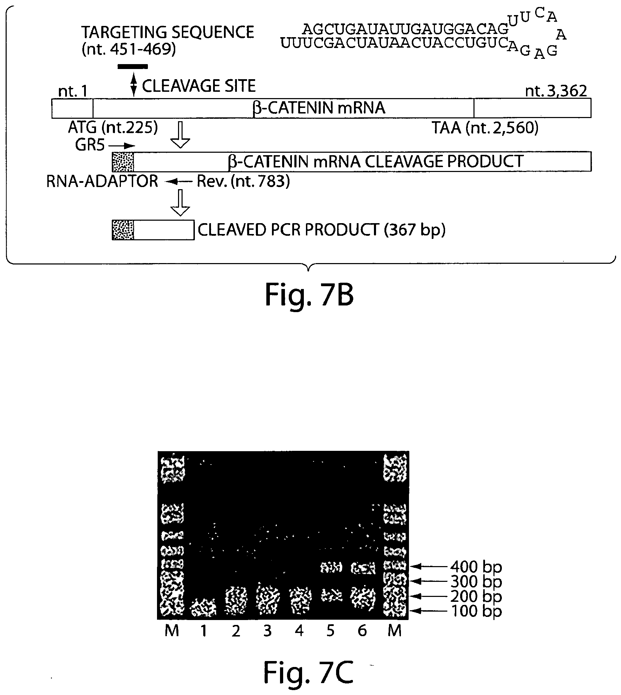

To further confirm that the *β-catenin* gene silencing is mediated specifically by shRNA, identification of the specific cleavage fragment of *β-catenin* mRNA was attempted by using 5'-RACE (rapid amplification of cDNA ends) PCR technique. A specific hallmark of RNAi-mediated gene silencing is the cleavage of *β*-*catenin* mRNA at the specific sites of the mRNA as predicted from the shRNA sequence. Based on the time course of *β-catenin* silencing (Fig. 7A), total RNA was isolated from SW 480 cells 8 h and 16 h after treatment with *E. coli* expressing shRNA against *β-catenin* to identify the cleaved fragments of mRNA. The cleaved *β-catenin* mRNA was found as early as 8 h after treatment with *E. coli* expressing shRNA: no fragments were detected in the control (Fig. 7B and 7C). The sequence analysis of the cleaved intermediate of *β-catenin* mRNA confirms the cleavage site located within the targeting sequence. This result shows that shRNA produced by bacteria trigger specific cleavage of the *β-catenin* mRNA through the RNAi-mediated gene silencing.

Induction of interferon response has been reported as a potential challenge to the specificity of some RNAi approaches (Bridge et al., Nat. Genet. 34, 263-264 (2003) and Hornung et al., Nat. Med. 11, 263-270 (2005)). To test if the gene silencing induced by the transkingdom RNAi is associated with interferon response induction, key interferon response genes were measured. The 2',5'-oligoadenlylate synthetases (OAS1 and OAS2) are important interferon-induced genes for the inhibition of cellular protein synthesis after viral infection. MX1 gene, a member of the interferon-induced myxovirus resistance protein family (MX proteins), participates in the innate host defense against RNA viruses. IFITM1, a member of the interferon-inducible transmembrane proteins, mediates the anti-proliferation activity of interferon. ISGF3γ is part of a cellular interferon receptor involved in interferon-induced transcription regulation and stimulation. These genes have been used as a standard panel for analyzing interferon response induction by interfering RNA (Interferon Response Detection Kit, SBI Systems Biosciences, CA). The mRNA of the five-interferon response genes were analyzed with semiquantitative RT-PCR. As shown in Fig. 7D, no induction of OAS1, OAS2, MX1, ISGF3γ and IFITM1 was detected following treatment with *E. coli* encoding shRNA against *β-catenin.* These data show that gene silencing induced by transkingdom RNAi is not associated with non-specific interferon response induction.

The mechanism of the transkingdom RNAi transfer was investigated. To determine if cellular entry of *E*. *coli* is required to induce RNAi, the gene-silencing activity of *E*. *coli* was compared with or without the *Inv* locus. The *Inv* encodes invasin that interacts with β1-integrin to facilitate the entry of *E*. *coli* into the cells. As expected, *E. coli* without *Inv* failed to enter cells (Fig. 8A). Surprisingly, *Inv* alone is not sufficient to enable *E. coli* to enter colon cancer cells (Fig. 8A), and no detectable gene silencing was observed in the absence of intracellular bacteria (Fig. 8B). The *Hly A* gene was introduced, which is thought to facilitate delivered genetic materials to escape from the entry vesicles (Grillot-Courvalin et al., Nat. Biotechnol. 16, 862-866 (1998). As expected, *Hly* alone failed to enable cell entrance of *E. coli,* but commensal *E. coli* with both *Inv* and *Hly* entered colon cancer cells with high efficiency (Fig. 8A). Under these conditions *β-catenin* was potently silenced up to 96 h (Fig. 8C). These results show that *E. coli* require both *Inv* and *Hly* to enter cells to induce transkingdom RNAi.

To determine whether gene silencing requires continued bacterial replication inside target cells, tetracycline was employed to block intracellular bacterial replication and gentamycin to remove extracellular bacteria. SW 480 cells were incubated with *E. coli* for 2 h followed by tetracycline treatment initiated at different times. As shown in Fig. 8D, following the initial 2 h infection time, an additional 2 h incubation time without tetracycline induced near maximum gene silencing; further delay in tetracycline treatment had no further enhancing effect on the degree of gene silencing. Surprisingly, there was no evidence of significant intracellular bacterial replication in the absence of tetracycline at 6 h and 48 h (Fig. 8E), which is likely due to the function of lysosomes and other intracellular antibacterial mechanisms (Roy et al., Science 304, 1515-1518 (2004) and Battistoni et al., Infect. Immun. 68, 30-37 (2000). These results show that transkingdom RNAi is not dependent on persistent bacterial replication inside target cells after the initial infection (2 h) and incubation time (2 h).

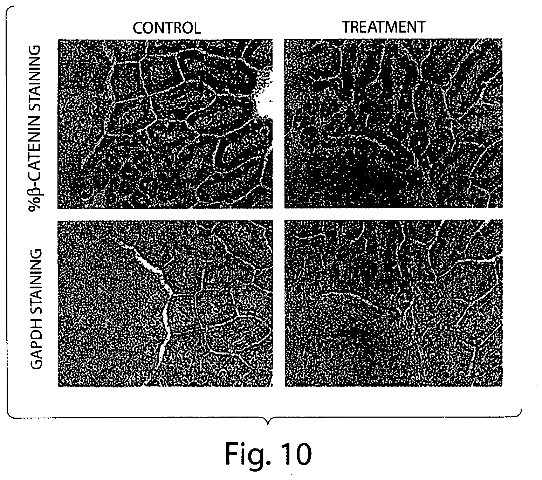

It was next determined if the transkingdom RNAi approach works *in vivo. E. coli* expressing shRNA against *β-catenin* were administered to mice orally. An inocculum of 5 x 10¹⁰ was administered orally five times per week, which is comparable to a human dosage of the probiotic *E. coli Nissle 1917.* Most of the inocculum is eliminated during passage through the bactericidal environment in the upper GI tract. Mice were treated with *E. coli* expressing shRNA against mouse *β-catenin* or with *E. coli* containing the corresponding plasmid vector. Treatment was continued for four weeks before the analysis of gene silencing by immunohistochemistry. As shown in Fig. 9A and 9B, *β-catenin* expression was silenced in the intestinal epithelium by *E. coli* expressing *β-catenin* shRNA (P< 0.01), not by the control *E. coli.* As a control, GAPDH expression was not reduced (Fig. 10). The gene silencing effect was more pronounced in the regions of or adjacent to the Peyer's patches (Fig. 9B). Treatment was well tolerated with no gross or microscopic signs of epithelial damage or ulcerations (Fig. 9B). These results show that mammals respond to *E. coli* expressing specific shRNA with powerful local RNAi *in vivo.*

The transkingdom RNAi approach was investigated to determine if it can be used to silence a disease gene after systemic dosing. Intravenous administration of therapeutic bacteria has been tested in clinical trials with demonstrated safety in cancer patients (Toso et al., J. Clin. Oncol. 20, 142-52 (2002)). Nude mice with xenografted human colon cancer were treated intravenously with of 10⁸ cfu of *E*. *coli* encoding shRNA against human *β-catenin.* Three doses were given at a 5-day interval. The treatments were well tolerated without adverse effects. As shown in Fig. 9, treatment with *E. coli* encoding shRNA against *β-catenin* resulted in significant decrease in *β-catenin* mRNA (p<0.005, Fig. 9C) and protein (p<0.01, Fig. 9D and 9E) in the tumor tissues. These data show that bacteria-mediated transkingdom RNAi can silence a disease gene in a distant part of the body after systemic administration.These results show that gene silencing can be achieved through a transkingdom system. Importantly, the potency and specificity of RNAi is preserved. Non-pathogenic *E. coli* has been used clinically as probiotics with demonstrated safety (Rembacken et al., Lancet 354, 635 (1999)). Therefore, this transkingdom system provides a practical and clinically compatible way to deliver RNA interference for medical indications. This *E*. *coli*-based RNAi technology also provides a convenient vector system for conducting RNAi-based functional studies of genes. Finally, the results invite an intriguing possibility that such exchange of interfering RNA may occur in nature under cohabitive, infectious, or symbiotic conditions.

### Example 5: In Vitro Assay of siRNAs Directed to TNFα Target Sequences

siRNAs were designed according to algorithm (Huesken D., et al. Design of a Genome-Wide siRNA Library Using an Artificial neural network. Nature Biotechnology 23 (8): 995-1001 (August 2005)) and cross-species reactivity, and screened in stimulated mouse macrophage RAW 264.7 and human macrophage THP-1 cells to determine if they are capable of silencing the TNFα target sequences illustrated in Tables 1-3. Nine mouse and human cross-specific siRNAs were tested in both the mouse and human cells, 36 human-specific siRNAs were tested in the human cells and 37 mouse-specific siRNAs were tested in the mouse cells.

### Material and Methods:

### Cell culture and transfection

The human monocytic cell line THP-1 was cultured in RPMI 1640 medium containing 2 mM L-glutamine, 1.5 g/L sodium bicarbonate, 4.5 g/L glucose, 10 mM HEPES, 1 mM sodium pyruvate, 10% heat-inactivated fetal bovine serum (FBS), with 100 U penicillin (pen) and 100 µg streptomycin (strep) per ml, and grown at 37°C in the presence of 5% CO2. Prior to transfection, cells were plated in complete medium into 96-well culture dishes (30,000 cells per well) and treated overnight with 162 nM phorbol 12-myristate 13-acetate (PMA) for differentiation into an adherent, macrophage-like form. To remove PMA and pen/strep, cells were washed once in 100 µl complete medium lacking pen/strep.

The murine macrophage cell line RAW264.7 was cultured in DMEM medium containing 4 mM L-glutamine, 1.5 g/L sodium bicarbonate, 4.5 g/L glucose, 10% heat-inactivated fetal bovine serum (FBS), with 100 U penicillin and 100 µg streptomycin per ml, and grown at 37°C in the presence of 5% CO2. Prior to transfection, cells were plated into 96-well culture dishes (30,000 cells per well) and allowed to adhere overnight. Cells were washed as above to remove penicillin and streptomycin.
In a separate 96-well dish, double-stranded RNAs for transfection were diluted into 50 µl Opti-MEM serum-free medium (Invitrogen) containing 0.3 µl Lipofectamine RNAiMAX (Invitrogen) and incubated 20 min at room temperature to enable the formation of transfection complexes. This mixture was then added to THP-1 or RAW cells (30-50% confluency), resulting in a final volume of 150 µl medium per well and a final RNA concentration of 50 nM. After 48 h, cells were treated with 0.1 µg/ml lipopolysaccharide (LPS) (Sigma) for 2 h to stimulate TNF-alpha production, and then harvested for RNA extraction.

RNA isolation, cDNA synthesis and quantitative real-time RT-PCR
RNA was prepared with the SV 96 Total RNA Isolation System (Promega) according to the manufacturer's protocol. cDNA was synthesized via the High Capacity cDNA Reverse Transcription Kit (Applied Biosystems) in a 20 µl reaction volume with 13.8 µl RNA, and used as template (8.8 µl per 25 ul reaction) in quantitative PCR performed with the Applied Biosystems (AB) 7500 Fast Real-Time PCR System. Reactions contained 1X GeneAmp Fast PCR Master Mix (Applied Biosystems), custom mouse or human TNF-alpha primers and TaqMan probes (400 nM each primer and 200 nM probe), and 1X commercial mouse or human GAPDH primers and probes (AB). Relative TNF-alpha abundance was calculated via the delta Ct method using GAPDH as an endogenous control.

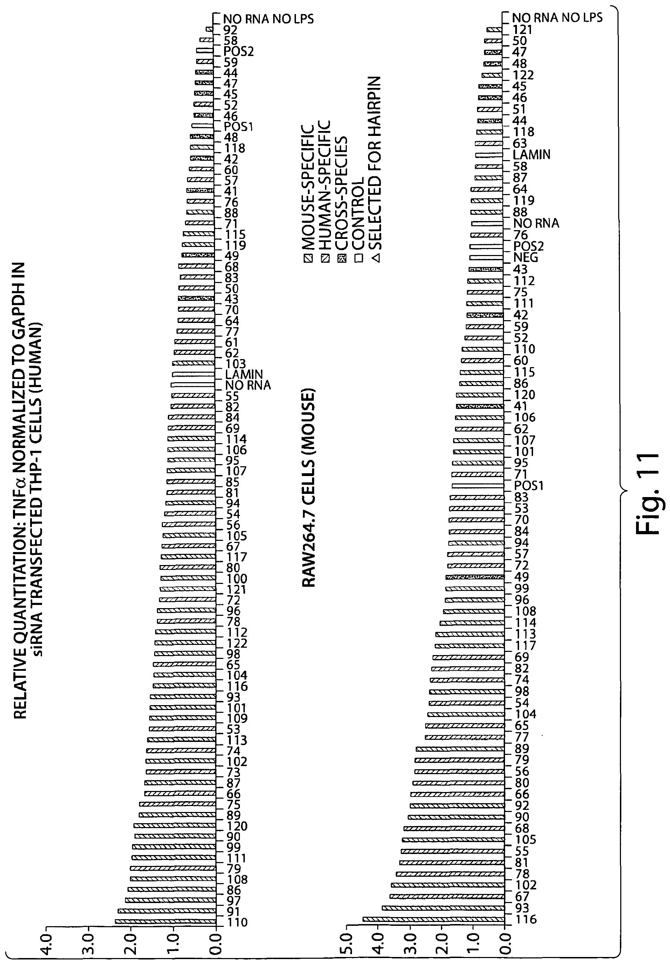

Each siRNA was screened twice and the following lead sequences were identified based upon the level of reduction of TNFα mRNA as determined by qRT-PCR. The data in Table 4 for mouse and Table 5 for Human shows the average of both experiments. Figure 11 illustrates the data in graphical format.

**Table 4.**

| **TNFα Target Sequence** | **Decrease of TNFα mRNA (%)** |
|---|---|
| CCCGAGTGACAAGCCTGTAGC (SEQ ID NO: 103) | 70 |
| AGGTCAACCTCCTCTCTGCCA (SEQ ID NO: 104) | 68 |
| ATCGCCCTACGGGTCATTGAG (SEQ ID NO: 105) | 51 |
| AATCGCCCTACGGGTCATTGA (SEQ ID NO: 106) | 47 |

**Table 5.**

| **TNFα Target Sequence** | **Decrease of TNFα mRNA (%)** |
|---|---|
| GAGTGACAAGCCTGTAGCCCA (SEQ ID NO: 107) | 72 |
| AGTGACAAGCCTGTAGCCCAT (SEQ ID NO: 108) | 68 |
| AGGTCAACCTCCTCTCTGCCA (SEQ ID NO: 109) | 60 |
| CCCGAGTGACAAGCCTGTAGC (SEQ ID NO: 110) | 48 |

## Claims

1. An ex vivo method of delivering one or more siRNAs to mammalian cells, the method comprising introducing to said mammalian cells at least one invasive bacterium containing one or more siRNAs or one or more DNA molecules encoding one or more siRNAs, wherein the siRNAs are targeted to any one of the TNFα target sequences of SEQ ID NOs: 1, 4, 5, 7, 81, or 82, and wherein the siRNAs interfere with the mRNA of TNFα.

2. An ex vivo method of regulating gene expression in mammalian cells, the method comprising introducing to said mammalian cells at least one invasive bacterium containing one or more siRNAs or one or more DNA molecules encoding one or more siRNAs, wherein the siRNAs are targeted to any one of the TNFα target sequences of SEQ ID NOs: 1, 4, 5, 7, 81, or 82, and wherein the siRNAs interfere with the mRNA of TNFα.

3. At least one invasive bacterium containing one or more siRNAs or one or more DNA molecules encoding one or more siRNAs, wherein the siRNAs are directed to any one of the TNFα target sequences of SEQ ID NOs: 1, 4, 5, 7, 81 or 82, and wherein the siRNAs interfere with the mRNA of TNFα for use in a method of treating or preventing an inflammatory disease or disorder in a mammal, the method comprising regulating the expression of at least one gene in a cell known to increase inflammation by introducing to the cells of the mammal said at least one invasive bacterium or said one or more DNA molecules.

4. The method of claims 1 or 2, or the invasive bacterium for use according to claim 3, wherein said invasive bacterium is
a) a non-pathogenic or non-virulent bacterium, or
b) a therapeutic bacterium.

5. The method of claims 1 or 2, or the invasive bacterium for use according to claim 3, wherein said mammalian cell is
a) selected from the group consisting of human, bovine, ovine, porcine, feline, buffalo, canine, goat, equine, donkey, deer, avian, bird, chicken and primate cells or
b) selected from the group consisting of a gastrointestinal epithelial cell and a macrophage.

6. The invasive bacterium for use according to claim 3, wherein said mammal is selected from the group consisting of human, bovine, ovine, porcine, feline, buffalo, canine, goat, equine, donkey, deer, avian, bird, chicken and primate.

7. The invasive bacterium for use according to claim 3, wherein the inflammatory disease or disorder is selected from the group consisting of inflammatory bowl disease, Crohn's disease, ulcerative colitis, rheumatoid arthritis and an allergy.

8. The method of claims 1 or 2, or the invasive bacterium for use according to claim 3, wherein said one or more DNA molecules encoding said one or more siRNAs
a) are transcribed within the animal cell, optionally wherein said one or more siRNAs are transcribed within the animal cell as shRNAs,
b) are transcribed within the bacterium, or
c) comprise a prokaryotic promoter optionally wherein said prokaryotic promoter is a T7 promoter.

9. The method of claims 1 or 2, or the invasive bacterium for use according to claim 3, wherein said animal cells are infected
a) with about 10³ to 10¹¹ viable invasive bacteria, optionally wherein said animal cells are infected with about 10⁵ to 10⁹ viable invasive bacteria, or
b) at a multiplicity of infection ranging from about 0.1 to 10⁶, optionally wherein said animal cells are infected at a multiplicity of infection ranging from about 10² to 10⁴.

10. The method of claims 1 or 2, or the invasive bacterium for use according to claim 3, wherein the siRNAs direct the RNA-induced silencing complex of the cell to interact with the mRNA of TNFα, optionally wherein said complex degrades said mRNA.

11. The method of claims 1 or 2, or the invasive bacterium for use according to claim 3, wherein expression of TNFα is decreased or inhibited.

## Patentansprüche

1. Ex-vivo-Verfahren zur Abgabe von einer oder mehr siRNAs an Säugetierzellen, wobei das Verfahren das Einführen von mindestens einem invasiven Bakterium in die Säugetierzellen umfasst, das eine oder mehr siRNAs oder ein oder mehr DNA-Moleküle beinhaltet, die eine oder mehr siRNAs kodieren, wobei die siRNAs auf eine der TNFα-Zielsequenzen von SEQ ID NO: 1, 4, 5, 7, 81, oder 82 abgezielt sind, und wobei die siRNAs mit der mRNA von TNFα interferieren.

2. Ex-vivo-Verfahren zur Regulation der Genexpression in Säugetierzellen, wobei das Verfahren das Einführen von mindestens einem invasiven Bakterium in die Säugetierzellen umfasst, das eine oder mehr siRNAs oder ein oder mehr DNA-Moleküle beinhaltet, die eine oder mehr siRNAs kodieren, wobei die siRNAs auf eine der TNFα-Zielsequenzen von SEQ ID NO: 1, 4, 5, 7, 8 1, oder 82 abgezielt sind, und wobei die siRNAs mit der mRNA von TNFα interferieren.

3. Mindestens ein invasives Bakterium, das eine oder mehr siRNAs oder ein oder mehr DNA-Moleküle umfasst, die eine oder mehr siRNAs kodieren, wobei die siRNAs auf eine der TNFα-Zielsequenzen von SEQ ID NO: 1, 4, 5, 7, 81, oder 82 abgezielt sind, und wobei die siRNAs mit der mRNA von TNFα interferieren, zur Verwendung in einem Verfahren der Behandlung oder Vorbeugung einer Entzündungserkrankung oder -störung in einem Säugetier, wobei das Verfahren die Regulation der Expression von mindestens einem Gen in einer Zelle, von dem bekannt ist, dass es die Entzündung verstärkt, durch Einführen von mindestens einem invasiven Bakterium oder dem einen oder den mehreren DNA-Molekülen in die Zellen der Säugetiere umfasst.

4. Verfahren nach den Ansprüchen 1 oder 2 oder invasives Bakterium zur Verwendung nach Anspruch 3, wobei das invasive Bakterium ist:
a) ein nicht-pathogenes oder nicht-virulentes Bakterium, oder
b) ein therapeutisches Bakterium.

5. Verfahren nach den Ansprüchen 1 oder 2 oder invasives Bakterium zur Verwendung nach Anspruch 3, wobei die Säugetierzelle
a) ausgewählt wird aus der Gruppe, bestehend aus Menschen-, Rinder-, Schafs-, Schweine-, Katzen-, Büffel-, Hunde-, Ziegen-, Pferde-, Esel-, Hirschen-, Geflügel-, Vogel-, Hühner- oder Primatenzelle, oder
b) ausgewählt wird aus der Gruppe, bestehend aus gastrointestinaler Epithelzelle und Makrophage.

6. Invasives Bakterium zur Verwendung nach Anspruch 3, wobei das Säugetier ausgewählt wird aus der Gruppe, bestehend aus Mensch, Rind, Schaf, Schwein, Katze, Büffel, Hund, Ziege, Pferd, Esel, Hirsch, Geflügel, Vogel, Huhn und Primat.

7. Invasives Bakterium zur Verwendung nach Anspruch 3, wobei die Entzündungserkrankung oder -störung ausgewählt wird aus der Gruppe, bestehend aus entzündlicher Darmerkrankung, Morbus Crohn, Colitis ulcerosa, rheumatoider Arthritis und einer Allergie.

8. Verfahren nach den Ansprüchen 1 oder 2 oder invasives Bakterium zur Verwendung nach Anspruch 3, wobei die einen oder mehreren DNA-Moleküle, die die eine oder mehreren siRNAs kodieren,
a) in der Tierzelle transkribiert werden, wobei die eine oder mehreren siRNAs in der Tierzelle wahlweise als shRNAs transkribiert werden,
b) im Bakterium transkribiert werden, oder
c) einen prokaryotischen Promotor umfassen, wobei der prokaryotische Promotor wahlweise ein T7-Promotor ist.

9. Verfahren nach den Ansprüchen 1 oder 2 oder invasives Bakterium zur Verwendung nach Anspruch 3, wobei die Tierzellen infiziert sind:
a) mit etwa 10³ bis 10¹¹ lebensfähigen invasiven Bakterien, wobei die Tierzellen wahlweise mit etwa 10⁵ bis 10⁹ lebensfähigen invasiven Bakterien infiziert sind, oder
b) bei einer Infektionsmultiplizität im Bereich von etwa 0,1 bis 10⁶, wobei die Tierzellen wahlweise bei einer Infektionsmultiplizität im Bereich von etwa 10² bis 10⁴ infiziert sind.

10. Verfahren nach den Ansprüchen 1 oder 2 oder invasives Bakterium zur Verwendung nach Anspruch 3, wobei die siRNAs den RNA-induzierten Silencing-Komplex der Zelle dazu anweisen, mit der mRNA eines TNFα zu interagieren, wobei der Komplex die mRNA wahlweise abbaut.

11. Verfahren nach den Ansprüchen 1 oder 2 oder invasives Bakterium zur Verwendung nach Anspruch 3, wobei die Expression von TNFα verringert oder gehemmt wird.

## Revendications

1. Procédé ex vivo permettant d'introduire un ou plusieurs ARNsi dans des cellules de mammifère, le procédé comprenant l'introduction dans lesdites cellules de mammifère d'au moins une bactérie invasive contenant un ou plusieurs ARNsi ou au moins une molécule d'ADN codant pour un ou plusieurs ARNsi ; dans lequel les ARNsi ciblent l'une quelconque des séquences cibles du TNFα des SEQ ID N° : 1, 4, 5, 7, 81 et 82, et les ARNsi interfèrent avec l'ARNm du TNFα.

2. Procédé ex vivo de régulation d'expression génique dans des cellules de mammifère, le procédé comprenant l'introduction dans lesdites cellules de mammifère d'au moins une bactérie invasive contenant un ou plusieurs ARNsi ou une ou plusieurs molécules d'ADN codant pour un ou plusieurs ARNsi, dans lequel les ARNsi ciblent l'une quelconque des séquences cibles du TNFα des SEQ ID N° : 1, 4, 5, 7, 81 et 82, et les ARNsi interfèrent avec l'ARNm du TNFα.

3. Au moins une bactérie invasive contenant un ou plusieurs ARNsi ou une ou plusieurs molécules d'ADN codant pour un ou plusieurs ARNsi, dans laquelle les ARNsi sont dirigés vers l'une quelconques des séquences cibles du TNFα des SEQ ID N° : 1, 4, 5, 7, 81 et 82, et les ARNsi interfèrent avec l'ARNm du TNFα, pour une utilisation dans une méthode de traitement ou de prévention d'une maladie ou d'un trouble inflammatoire chez un mammifère, la méthode comprenant la régulation de l'expression d'au moins un gène dans une cellule, connue pour accroitre l'inflammation, par introduction de ladite au moins une bactérie invasive ou desdites une ou plusieurs molécules d'ADN dans les cellules du mammifère.

4. Procédé selon la revendication 1 ou 2, ou bactérie invasive destinée à une utilisation selon la revendication 3, laquelle bactérie invasive étant
a) une bactérie non pathogène ou non virulente, ou
b) une bactérie thérapeutique.

5. Procédé selon la revendication 1 ou 2, ou bactérie invasive destinée à une utilisation selon la revendication 3, ladite cellule de mammifère étant choisie
a) dans le groupe constitué par les cellules humaines, bovines, ovines, porcines, félines, de bison, canines, caprines, équines, d'âne, de cervidés, aviaires, d'oiseau, de poule et de primate, ou
b) dans le groupe constitué par une cellule épithéliale gastro-intestinale et un macrophage.

6. Bactérie invasive destinée à une utilisation selon la revendication 3, dans laquelle ledit mammifère est choisi dans le groupe constitué par les humains, les bovidés, les ovidés, les porcidés, les félidés, les bisons, les canidés, les capridés, les équidés, les ânes, les cervidés, les volatiles, les oiseaux, les poules et les primates.

7. Bactérie invasive destinée à une utilisation selon la revendication 3, dans laquelle la maladie ou le trouble inflammatoire est choisi dans le groupe constitué par la maladie inflammatoire chronique de l'intestin, la maladie de Crohn, la rectocolite hémorragique, la polyarthrite rhumatoïde et une allergie.

8. Procédé selon la revendication 1 ou 2, ou bactérie invasive destinée à une utilisation selon la revendication 3, dans lesquels lesdites une ou plusieurs molécules d'ADN codant pour lesdits un ou plusieurs ARNsi
a) sont transcrites dans la cellule animale, lesdits un ou plusieurs ARNsi étant éventuellement transcrits dans la cellule animale sous la forme d'ARNsh,
b) sont transcrites dans la bactérie, ou
c) comprennent un promoteur procaryote, lequel promoteur procaryote consiste éventuellement en un promoteur T7.

9. Procédé selon la revendication 1 ou 2, ou bactérie invasive destinée à une utilisation selon la revendication 3, lesdites cellules animales étant infectées
a) par environ 10³ à 10¹¹ bactéries invasives viables, les cellules animales étant éventuellement infectées par environ 10⁵ à 10⁹ bactéries invasives viables, ou
b) à une multiplicité d'infection située dans une plage allant d'environ 0,1 à 10⁶, les cellules animales étant éventuellement infectées à une multiplicité d'infection située dans une plage allant d'environ 10² à 10⁴.

10. Procédé selon la revendication 1 ou 2, ou bactérie invasive destinée à une utilisation selon la revendication 3, dans lesquels les ARNsi dirigent le complexe de silençage induit par ARN de la cellule afin qu'il interagisse avec l'ARNm du TNFα, ledit complexe dégradant éventuellement ledit ARNm.

11. Procédé selon la revendication 1 ou 2, ou bactérie invasive destinée à une utilisation selon la revendication 3, dans lesquels l'expression du TNFα est réduite ou inhibée.
